# EUROPEAN PATENT APPLICATION

(11) **EP 4 198 104 A1**
(43) Date of publication of application: **21.06.2023**
(21) Application number: 21858253.4
(22) Date of filing: 13.08.2021
(51) Int. Cl.: C09K 11/06, H01L 51/50

(54) **ORGANIC ELECTROLUMINESCENT ELEMENT**

(30) Priority: 17.08.2020 JP 2020137612
(71) Applicant: Hodogaya Chemical Co., Ltd., Tokyo, 105-0021 (JP); SFC Co., Ltd., Chungcheongbuk-do (KR)
(72) Inventor: IZUMIDA, Junichi, Tokyo 105-0021 (JP); KO, Sang-Won, Tokyo 105-0021 (JP); LEE, Bong-Hyang, Tokyo 105-0021 (JP); RYU, Jung-Ho, Tokyo 105-0021 (JP); YAMAMOTO, Takeshi, Tokyo 105-0021 (JP); SURUGA, Kazuyuki, Tokyo 105-0021 (JP); CHA, Soon-Wook, Cheongju-si, Chungcheongbuk-do (KR); JOO, Sung-Hoon, Cheongju-si, Chungcheongbuk-do (KR); YANG, Byung-Sun, Cheongju-si, Chungcheongbuk-do (KR); KIM, Ji-Hwan, Cheongju-si, Chungcheongbuk-do (KR)
(74) Representative: Müller-Boré & Partner Patentanwälte PartG mbB
(86) International application number: PCT/JP2021/029801
(87) International publication number: WO 2022/039106

(57) **Abstract**

[Problem]

An object of the present invention is to provide a material for an organic EL device having excellent hole injection and transport performance, electron blocking ability, stability in a thin film state, and durability, and further to provide an organic EL device having high efficiency, a low driving voltage, and a long lifetime by combining the material with various materials for an organic EL device having excellent hole and electron injection and transport performance, electron blocking ability, stability in a thin film state, and durability in such a manner that the characteristics of each of the materials can be effectively exhibited.

[Solution]

An organic EL device was fabricated by focusing on the fact that a triarylamine compound having a specific structure has excellent hole injection and transport ability, thin film stability, and durability, and selecting a specific arylamine compound as a material for forming a second hole transport layer, and the invention was completed.

## Description

### Technical Field

The present invention relates to an organic electroluminescent device which is a self-luminous device suitable for various display devices and specifically relates to an organic electroluminescent device (hereinafter abbreviated as organic EL device) using a specific arylamine compound.

### Background Art

The organic EL device is a self-luminous device, and therefore is brighter and has better visibility than a liquid crystal device, and enables a clear display, and thus has been actively studied.

In 1987, C. W. Tang et al. at Eastman Kodak developed a laminated structure device using materials assigned with different roles, thereby realizing practical applications of an organic EL device with organic materials. They laminated a phosphor capable of transporting electrons and an organic substance capable of transporting holes, and injected both charges into a phosphor layer to cause emission so as to obtain a high luminance of 1,000 cd/m² or more at a voltage of 10 V or less (see, for example, PTLs 1 and 2).

To date, many improvements have been made for practical applications of the organic EL device, and high efficiency and durability have come to be realized by an electroluminescent device in which various roles of the laminated structure are further subdivided, and an anode, a hole injection layer, a hole transport layer, a light emitting layer, an electron transport layer, an electron injection layer, and a cathode are sequentially provided on a substrate (see, for example, NPL 1).

Further, there have been attempts to use triplet excitons for further improvements of luminous efficiency, and the use of a phosphorescent compound has been examined (see, for example, NPL 2).

Then, devices that use light emission caused by thermally activated delayed fluorescence (TADF) have also been developed. In 2011, Adachi et al. at Kyushu University achieved an external quantum efficiency of 5.3% with a device using a thermally activated delayed fluorescent material (see, for example, NPL 3).

The light emitting layer can be also fabricated by doping a charge transporting compound generally called a host material with a fluorescent compound, a phosphorescent compound, or a material that emits delayed fluorescence. As described in the Non Patent Literature, the selection of an organic material in an organic EL device greatly affects various characteristics such as efficiency and durability of the device (see, for example, NPLs 1 to 3).

In an organic EL device, charges injected from both electrodes recombine in a light emitting layer to cause emission. What is important here is how efficiently the hole and electron charges are transferred to the light emitting layer, and it is necessary to form a device having excellent carrier balance. In addition, the probability of hole-electron recombination can be improved by enhancing hole injectability and electron blocking performance of blocking electrons injected from the cathode, and moreover, high luminous efficiency can be obtained by confining excitons generated in the light emitting layer. Therefore, the role of a hole transport material is important, and there has been a demand for a hole transport material that has high hole injectability, large hole mobility, high electron blocking performance, and high durability to electrons.

Heat resistance and amorphousness of materials are also important for the lifetime of the device. In a material with low heat resistance, thermal decomposition occurs even at a low temperature due to heat generated during driving of the device, which leads to deterioration of the material. In a material with low amorphousness, crystallization of a thin film occurs even in a short time, which leads to deterioration of the device. Therefore, the material to be used is required to have characteristics of high heat resistance and favorable amorphousness.

N,N'-diphenyl-N,N'-di(α-naphthyl)benzidine (NPD) and various aromatic amine derivatives are known as the hole transport materials having been used in the organic EL device so far (see, for example, PTLs 1 and 2). Although NPD has favorable hole transport ability, its glass transition point (Tg) which can serve as an index of heat resistance is as low as 96°C, which causes deterioration of device characteristics by crystallization under a high temperature condition (see, for example, NPL 4). Further, among the aromatic amine derivatives described in the Patent Literature, a compound having an excellent hole mobility of 10⁻³ cm²/Vs or more is known (see, for example, PTLs 1 and 2). However, since the electron blocking performance is insufficient, some electrons pass through the light emitting layer, and improvements in luminous efficiency cannot be expected, and so on, and thus, a material with higher electron blocking performance, a more stable as a thin film, and higher heat resistance has been demanded for higher efficiency. Although an aromatic amine derivative having high durability is reported (see, for example, PTL 3), the derivative is used as a charge transport material used in an electrophotographic photoconductor, and there is no example of using the derivative as the organic EL device.

An arylamine compound having a substituted carbazole structure is proposed as a compound having improved characteristics such as heat resistance and hole injectability (see, for example, PTLs 4 and 5). However, while in a device using such a compound for the hole injection layer or the hole transport layer, heat resistance, luminous efficiency, and the like have been improved, the improvements are still insufficient, and a further decrease in driving voltage and a further increase in luminous efficiency have been demanded.

In order to improve characteristics of the organic EL device and to improve the yield of fabrication of the device, there has been a demand for a device that can recombine holes and electrons with high efficiency, and has high luminous efficiency, a low driving voltage, and a long lifetime by combining materials having excellent hole and electron injection and transport performance, stability as a thin film, and durability.

Further, in order to improve characteristics of the organic EL device, there has been a demand for a device that maintains carrier balance and has high efficiency, a low driving voltage, and a long lifetime by combining materials having excellent hole and electron injection and transport performance, stability as a thin film, and durability.

### Citation List

### Patent Literature

PTL 1: JPH08-048656A
PTL 2: JP3194657B
PTL 3: JP4943840B
PTL 4: JP2006-151979A
PTL 5: WO2008/062636
PTL 6: WO2014/009310

### Non Patent Literature

NPL 1: Proceedings of the 9th Meeting of the Japan Society of Applied Physics, pp. 55 to 61 (2001)
NPL 2: Proceedings of the 9th Meeting of the Japan Society of Applied Physics, pp. 23 to 31 (2001)
NPL 3: Appl. Phys. Let., 98, 083302 (2011)
NPL 4: Proceedings of the 3rd Meeting of the Japan OLED Forum, pp. 13 to 14 (2006)

### Summary of Invention

### Technical Problem

An object of the invention is to provide a material for an organic EL device having excellent hole injection and transport performance, electron blocking ability, stability in a thin film state, and durability as a material for an organic EL device having high efficiency and high durability, and also to provide an organic EL device having high efficiency, a low driving voltage, and a long lifetime by combining the material with various materials for an organic EL device having excellent hole and electron injection and transport performance, electron blocking ability, stability in a thin film state, and durability in such a manner that the characteristics of each of the materials can be effectively exhibited.

Physical properties that the organic compound to be provided by the invention should have include (1) good hole injection characteristics, (2) large hole mobility, (3) an excellent electron blocking ability, (4) stability in a thin film state, and (5) excellent heat resistance. In addition, physical properties that the organic EL device to be provided by the invention should have include (1) high luminous efficiency and high power efficiency, (2) a low turn on voltage, (3) a low actual driving voltage, and (4) a long lifetime.

### Solution to Problem

In order to achieve the object, the present inventors conducted intensive studies, and as a result, they found that an arylamine compound having a specific structure has excellent hole injection and transport ability, stability as a thin film, and durability, and therefore, when such a compound is selected as a material of a hole transport layer, holes injected from the anode side can be efficiently transported. Furthermore, the present inventors fabricated various organic EL devices combined with light emitting materials having a specific structure and the like, and evaluated the characteristics of the devices, and as a result, they completed the invention.

That is, according to the invention, the following organic EL devices are provided.
1) An organic electroluminescent device (organic EL device) including at least an anode, a first hole transport layer, a second hole transport layer, a light emitting layer, an electron transport layer, and a cathode in this order, characterized in that the second hole transport layer contains an arylamine compound represented by the following general formula (1). 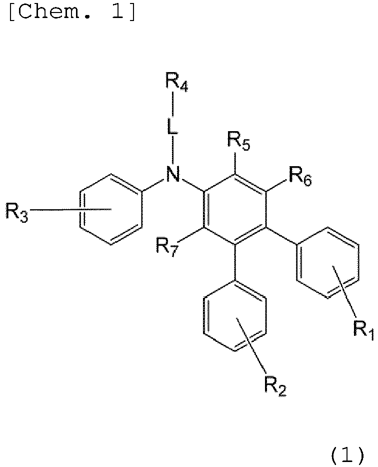 In the general formula (1), R₁ to R₄ may be the same or different, and represent a hydrogen atom, a deuterium atom, a carbonyl group, a cyano group, a silyl group which may have a substituent, a phosphino group which may have a substituent, a phosphine oxide group which may have a substituent, a linear or branched alkyl group having 1 to 6 carbon atoms which may have a substituent, or a substituted or unsubstituted aromatic hydrocarbon group having 6 to 25 ring-forming carbon atoms; L represents a divalent group of a substituted or unsubstituted aromatic hydrocarbon having 6 to 20 ring-forming carbon atoms or a single bond; and R₅ to R₇ represent a hydrogen atom, a deuterium atom, a linear or branched alkyl group having 1 to 6 carbon atoms which may have a substituent, a halogen atom, or a cyano group.
2) The organic EL device described in the above 1), characterized in that in the formula (1), R₁ and R₂ may be the same or different, a hydrogen atom, a silyl group which may have a substituent, or a substituted or unsubstituted aromatic hydrocarbon group having 6 to 20 ring-forming carbon atoms; R₃ may be a silyl group which may have a substituent or a substituted or unsubstituted aromatic hydrocarbon group having 6 to 25 ring-forming carbon atoms; and R₄ may be a hydrogen atom, a silyl group which may have a substituent, or a substituted or unsubstituted aromatic hydrocarbon group having 6 to 25 ring-forming carbon atoms.
3) The organic EL device described in the above 1) or 2), characterized in that in the formula (1), R₁ and R₂ may be the same or different, any of a hydrogen atom, an unsubstituted phenyl group, an unsubstituted naphthyl group, an unsubstituted biphenylyl group, a phenyl group substituted with a naphthyl group, and an unsubstituted terphenylyl group; R₃ may be any of an unsubstituted biphenylyl group, a phenyl group substituted with a naphthyl group, a phenyl group substituted with two phenyl groups, and a naphthyl group substituted with a phenyl group; and R₄ may be any of an unsubstituted phenyl group, an unsubstituted naphthyl group, and a naphthyl group substituted with a phenyl group.
4) The organic EL device described in any one of the above 1) to 3), characterized in that in the formula (1), R₁ may be any of an unsubstituted phenyl group, an unsubstituted biphenylyl group, a phenyl group substituted with a naphthyl group, and an unsubstituted terphenylyl group; R₂ may be any of a hydrogen atom, an unsubstituted phenyl group, an unsubstituted naphthyl group, an unsubstituted biphenylyl group, a phenyl group substituted with a naphthyl group, and an unsubstituted terphenylyl group; R₃ may be an unsubstituted phenyl group; and R₄ may be any of an unsubstituted phenyl group, an unsubstituted naphthyl group, and a naphthyl group substituted with a phenyl group.
5) The organic EL device described in the above 1) or 2), characterized in that in the formula (1), at least one of R₁ to R₄ is either a triphenylsilyl group or a phenyl group substituted with a triphenylsilyl group.
6) The organic EL device described in any one of the above 1) to 5), characterized in that in the formula (1), all of R₅ to R₇ are a hydrogen atom.
7) The organic EL device described in any one of the above 1) to 6), characterized in that in the formula (1), L is a 1,4-phenylene group.
8) The organic EL device described in any one of the above 1) to 7), characterized in that the blue light emitting layer contains a pyrene derivative having a pyrene skeleton in the molecule as a blue light emitting dopant.
9) The organic EL device described in any one of the above 1) to 7), characterized in that the blue light emitting layer contains a compound represented by the following general formula (2) or general formula (3) as a blue light emitting dopant. 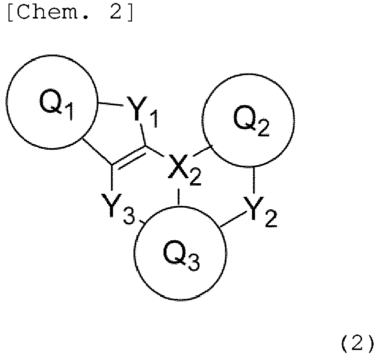 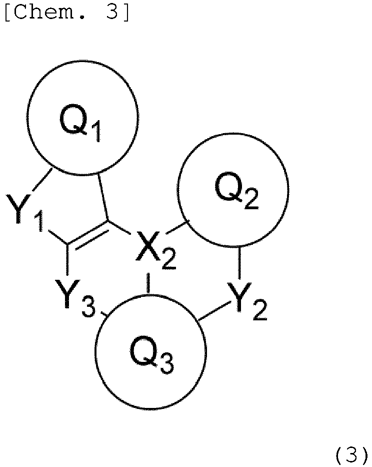 In the general formula (2) and the general formula (3), Q₁ to Q₃ may be the same or different, and represent a substituted or unsubstituted aromatic hydrocarbon or a substituted or unsubstituted aromatic heterocyclic ring; X₂ represents B, P, P=O, or P=S; Y₁ to Y₃ may be the same or different, and represent any one selected from N-R₈, CR₉R₁₀, O, S, Se, and SiR₁₁R₁₂, R₈ to R₁₂ may be the same or different, and represent a hydrogen atom, a deuterium atom, a fluorine atom, a chlorine atom, a cyano group, a nitro group, a linear or branched alkyl group having 1 to 6 carbon atoms which may have a substituent, a cycloalkyl group having 5 to 10 carbon atoms which may have a substituent, a linear or branched alkenyl group having 2 to 6 carbon atoms which may have a substituent, a linear or branched alkyloxy group having 1 to 6 carbon atoms which may have a substituent, a cycloalkyloxy group having 5 to 10 carbon atoms which may have a substituent, a substituted or unsubstituted aromatic hydrocarbon group, a substituted or unsubstituted aromatic heterocyclic group, or a substituted or unsubstituted aryloxy group, R₉ and R₁₀, and R₁₁ and R₁₂ may bind to each other to form a ring via a single bond, a substituted or unsubstituted methylene group, an oxygen atom, a sulfur atom, or a monosubstituted amino group, provided that when Y₁ to Y₃ are N-R₈, CR₉R₁₀, or SiR₁₁R₁₂, R₈ to R₁₂ may each bind to the adjacent Q₁ to Q₃ to form a ring via a single bond or a linking group such as a substituted or unsubstituted methylene group, an oxygen atom, a sulfur atom, or a monosubstituted amino group.
10) The organic EL device described in any one of the above 1) to 9), characterized in that the blue light emitting layer contains an anthracene derivative having an anthracene skeleton in the molecule.

Specific examples of the "linear or branched alkyl group having 1 to 6 carbon atoms" in the "linear or branched alkyl group having 1 to 6 carbon atoms which may have a substituent" represented by R₁ to R₇ in the general formula (1) include a methyl group, an ethyl group, an n-propyl group, an isopropyl group, an n-butyl group, an isobutyl group, a tert-butyl group, an n-pentyl group, an isopentyl group, a neopentyl group, and an n-hexyl group.

Specific examples of the "aromatic hydrocarbon group having 6 to 25 carbon atoms" in the "substituted or unsubstituted aromatic hydrocarbon group having 6 to 25 ring-forming carbon atoms" represented by R₁ to R₄ in the general formula (1) include a phenyl group, a biphenylyl group, a terphenylyl group, a naphthyl group, an anthracenyl group, a phenanthrenyl group, a fluorenyl group, an indenyl group, a pyrenyl group, a perylenyl group, a fluoranthenyl group, and a triphenylenyl group.

Among the groups exemplified above, the aromatic hydrocarbon group having 6 to 20 ring-forming carbon atoms can be exemplified as a group defined as the "aromatic hydrocarbon group having 6 to 20 ring-forming carbon atoms".

Specific examples of the "substituent" in the "linear or branched alkyl group having 1 to 6 carbon atoms and having a substituent" represented by R₁ to R₇ in the general formula (1) include a deuterium atom, a cyano group, a nitro group; halogen atoms such as a fluorine atom, a chlorine atom, a bromine atom, and an iodine atom; linear or branched alkyloxy groups having 1 to 6 carbon atoms such as a methyloxy group, an ethyloxy group, and a propyloxy group; alkenyl groups such as a vinyl group and an allyl group; aryloxy groups such as a phenyloxy group and a tolyloxy group; arylalkyloxy groups such as a benzyloxy group and a phenethyloxy group; aromatic hydrocarbon groups such as a phenyl group, a biphenylyl group, a terphenylyl group, a naphthyl group, an anthracenyl group, a phenanthrenyl group, a fluorenyl group, an indenyl group, a pyrenyl group, a perylenyl group, a fluoranthenyl group, and a triphenylenyl group; aromatic heterocyclic groups such as a pyridyl group, a pyrimidinyl group, a triazinyl group, a thienyl group, a furyl group, a pyrrolyl group, a quinolyl group, an isoquinolyl group, a benzofuranyl group, a benzothienyl group, an indolyl group, a carbazolyl group, a benzoxazolyl group, a benzothiazolyl group, a quinoxalinyl group, a benzimidazolyl group, a pyrazolyl group, a dibenzofuranyl group, a dibenzothienyl group, and a carbolinyl group; disubstituted amino groups substituted with an aromatic hydrocarbon group such as a diphenylamino group and a dinaphthylamino group; disubstituted amino groups substituted with an aromatic heterocyclic group such as a dipyridylamino group and a dithienylamino group; and disubstituted amino groups substituted with a substituent selected from an aromatic hydrocarbon group or an aromatic heterocyclic group, and these substituents may be further substituted with a substituent exemplified above.

Specific examples of the "substituent" in the "silyl group having a substituent", the "phosphino group having a substituent", the "phosphine oxide group having a substituent", or the "aromatic hydrocarbon group having 6 to 25 ring-forming carbon atoms and having a substituent" represented by R₁ to R₄ in the general formula (1) include a deuterium atom, a cyano group, a nitro group; halogen atoms such as a fluorine atom, a chlorine atom, a bromine atom, and an iodine atom; linear or branched alkyl groups having 1 to 6 carbon atoms such as a methyl group, an ethyl group, an n-propyl group, an isopropyl group, an n-butyl group, an isobutyl group, a tert-butyl group, an n-pentyl group, an isopentyl group, a neopentyl group, and an n-hexyl group; linear or branched alkyloxy groups having 1 to 6 carbon atoms such as a methyloxy group, an ethyloxy group, and a propyloxy group; alkenyl groups such as a vinyl group and an allyl group; aryloxy groups such as a phenyloxy group and a tolyloxy group; arylalkyloxy groups such as a benzyloxy group and a phenethyloxy group; aromatic hydrocarbon groups such as a phenyl group, a biphenylyl group, a terphenylyl group, a naphthyl group, an anthracenyl group, a phenanthrenyl group, a fluorenyl group, an indenyl group, a pyrenyl group, a perylenyl group, a fluoranthenyl group, and a triphenylenyl group; aromatic heterocyclic groups such as a pyridyl group, a pyrimidinyl group, a triazinyl group, a thienyl group, a furyl group, a pyrrolyl group, a quinolyl group, an isoquinolyl group, a benzofuranyl group, a benzothienyl group, an indolyl group, a carbazolyl group, a benzoxazolyl group, a benzothiazolyl group, a quinoxalinyl group, a benzimidazolyl group, a pyrazolyl group, a dibenzofuranyl group, a dibenzothienyl group, and a carbolinyl group; disubstituted amino groups substituted with an aromatic hydrocarbon group such as a diphenylamino group and a dinaphthylamino group; disubstituted amino groups substituted with an aromatic heterocyclic group such as a dipyridylamino group and a dithienylamino group; and disubstituted amino groups substituted with a substituent selected from an aromatic hydrocarbon group or an aromatic heterocyclic group, and these substituents may be further substituted with a substituent exemplified above.

Specific examples of the "aromatic hydrocarbon having 6 to 20 ring-forming carbon atoms" of the "substituted or unsubstituted aromatic hydrocarbon having 6 to 20 ring-forming carbon atoms" in the "divalent group of a substituted or unsubstituted aromatic hydrocarbon having 6 to 20 ring-forming carbon atoms" represented by L in the general formula (1) include benzene, biphenyl, terphenyl, naphthalene, anthracene, acenaphthalene, fluorene, phenanthrene, indane, pyrene, and triphenylene.

Then, the "divalent group of an aromatic hydrocarbon having 6 to 20 ring-forming carbon atoms" in the "divalent group of a substituted or unsubstituted aromatic hydrocarbon having 6 to 20 ring-forming carbon atoms" represented by L in the general formula (1) denotes a divalent group obtained by removing two hydrogen atoms from the above "aromatic hydrocarbon having 6 to 20 ring-forming carbon atoms".

Specific examples of the "substituent" in the "divalent group of an aromatic hydrocarbon having 6 to 20 ring-forming carbon atoms and having a substituent" represented by L in the general formula (1) include a deuterium atom, a cyano group, a nitro group; halogen atoms such as a fluorine atom, a chlorine atom, a bromine atom, and an iodine atom; linear or branched alkyl groups having 1 to 6 carbon atoms such as a methyl group, an ethyl group, an n-propyl group, an isopropyl group, an n-butyl group, an isobutyl group, a tert-butyl group, an n-pentyl group, an isopentyl group, a neopentyl group, and an n-hexyl group; linear or branched alkyloxy groups having 1 to 6 carbon atoms such as a methyloxy group, an ethyloxy group, and a propyloxy group; alkenyl groups such as a vinyl group and an allyl group; aryloxy groups such as a phenyloxy group and a tolyloxy group; arylalkyloxy groups such as a benzyloxy group and a phenethyloxy group; aromatic hydrocarbon groups such as a phenyl group, a biphenylyl group, a terphenylyl group, a naphthyl group, an anthracenyl group, a phenanthrenyl group, a fluorenyl group, an indenyl group, a pyrenyl group, a perylenyl group, a fluoranthenyl group, and a triphenylenyl group; aromatic heterocyclic groups such as a pyridyl group, a pyrimidinyl group, a triazinyl group, a thienyl group, a furyl group, a pyrrolyl group, a quinolyl group, an isoquinolyl group, a benzofuranyl group, a benzothienyl group, an indolyl group, a carbazolyl group, a benzoxazolyl group, a benzothiazolyl group, a quinoxalinyl group, a benzimidazolyl group, a pyrazolyl group, a dibenzofuranyl group, a dibenzothienyl group, and a carbolinyl group; disubstituted amino groups substituted with an aromatic hydrocarbon group such as a diphenylamino group and a dinaphthylamino group; disubstituted amino groups substituted with an aromatic heterocyclic group such as a dipyridylamino group and a dithienylamino group; and disubstituted amino groups substituted with a substituent selected from an aromatic hydrocarbon group or an aromatic heterocyclic group, and these substituents may be further substituted with a substituent exemplified above.

In the general formula (1), R₁ is preferably a "hydrogen atom", a "silyl group which may have a substituent", or a "substituted or unsubstituted aromatic hydrocarbon group having 6 to 20 ring-forming carbon atoms", more preferably a "hydrogen atom", a "substituted or unsubstituted phenyl group", an "unsubstituted naphthyl group", an "unsubstituted biphenylyl group", an "unsubstituted terphenylyl group", or a "triphenylsilyl group", and still more preferably a "hydrogen atom", an "unsubstituted phenyl group", an "unsubstituted naphthyl group", or an "unsubstituted biphenylyl group".

Here, the "substituent" of the "phenyl group having a substituent" is preferably a "phenyl group", a "biphenyl group", a "naphthyl group", or a "triphenylsilyl group".

Specifically, R₁ is more preferably a "hydrogen atom", an "unsubstituted phenyl group", an "unsubstituted naphthyl group", an "unsubstituted biphenylyl group", an "unsubstituted terphenylyl group", a "phenyl group substituted with a naphthyl group", a "triphenylsilyl group", or a "phenyl group substituted with a triphenylsilyl group", and still more preferably a "hydrogen atom", an "unsubstituted phenyl group", an "unsubstituted naphthyl group", or an "unsubstituted biphenylyl group".

In the general formula (1), R₂ is preferably a "hydrogen atom", a "silyl group which may have a substituent", or a "substituted or unsubstituted aromatic hydrocarbon group having 6 to 20 ring-forming carbon atoms", more preferably a "hydrogen atom", a "substituted or unsubstituted phenyl group", an "unsubstituted naphthyl group", an "unsubstituted biphenylyl group", an "unsubstituted terphenylyl group", or a "triphenylsilyl group", and still more preferably a "hydrogen atom" or an "unsubstituted phenyl group".

Here, the "substituent" of the "phenyl group having a substituent" is preferably a "phenyl group", a "biphenyl group", a "naphthyl group", or a "triphenylsilyl group".

Specifically, R₂ is more preferably a "hydrogen atom", an "unsubstituted phenyl group", an "unsubstituted naphthyl group", an "unsubstituted biphenylyl group", an "unsubstituted terphenylyl group", a "phenyl group substituted with a naphthyl group", a "triphenylsilyl group", or a "phenyl group substituted with a triphenylsilyl group", and still more preferably a "hydrogen atom" or an "unsubstituted phenyl group".

In the general formula (1), R₃ is preferably a "silyl group which may have a substituent" or a "substituted or unsubstituted aromatic hydrocarbon group having 6 to 25 ring-forming carbon atoms", more preferably a "substituted or unsubstituted phenyl group", a "substituted or unsubstituted biphenyl group", a "substituted or unsubstituted naphthyl group", or a "triphenylsilyl group", and still more preferably a "substituted or unsubstituted phenyl group", an "unsubstituted biphenylyl group", or a "naphthyl group having a substituent".

Here, the "substituent" of the "phenyl group having a substituent", the "biphenyl group having a substituent", or the "naphthyl group having a substituent" is preferably a "phenyl group", a "naphthyl group", or a "triphenylsilyl group", and more preferably a "phenyl group" or a "naphthyl group". In addition, it is also preferred to include a plurality of (for example, two) "phenyl groups".

Specifically, R₃ is more preferably an "unsubstituted phenyl group", an "unsubstituted biphenyl group", a "naphthyl group having a substituent", a "triphenylsilyl group", or a "phenyl group substituted with a triphenylsilyl group", and still more preferably an "unsubstituted phenyl group", an "unsubstituted biphenylyl group", a "phenyl group substituted with a naphthyl group", a "phenyl group substituted with two phenyl groups", or a "naphthyl group substituted with a phenyl group".

In the general formula (1), R₄ is preferably a "hydrogen atom", a "silyl group which may have a substituent", or a "substituted or unsubstituted aromatic hydrocarbon group having 6 to 25 ring-forming carbon atoms", more preferably an "unsubstituted phenyl group", a "substituted or unsubstituted naphthyl group", or a "triphenylsilyl group", and still more preferably a "substituted or unsubstituted naphthyl group".

Here, the "substituent" of the "naphthyl group having a substituent" is preferably a "phenyl group" or a "triphenylsilyl group", and more preferably a "phenyl group".

Specifically, R₄ is more preferably an "unsubstituted phenyl group", an "unsubstituted naphthyl group", a "naphthyl group substituted with a phenyl group", a "triphenylsilyl group", or a "phenyl group substituted with a triphenylsilyl group", and still more preferably an "unsubstituted naphthyl group" or a "naphthyl group substituted with a phenyl group".

In the general formula (1), it is preferred that at least one of R₁ to R₄ is a triphenylsilyl group or a phenyl group substituted with a triphenylsilyl group.

Further, it is preferred that R₅ to R₇ are a hydrogen atom, and it is more preferred that all of R₅ to R₇ are a hydrogen atom.

In the general formula (1), L is preferably a "divalent group of a substituted or unsubstituted aromatic hydrocarbon having 6 to 20 ring-forming carbon atoms", more preferably a divalent group obtained by removing two hydrogen atoms from benzene, biphenyl, or naphthalene, and still more preferably a 1,4-phenylene group.

Specific examples of the "aromatic hydrocarbon" or the "aromatic heterocyclic ring" in the "substituted or unsubstituted aromatic hydrocarbon", or the "substituted or unsubstituted aromatic heterocyclic ring" represented by Q₁ to Q₃ in the general formula (2) and the general formula (3) include benzene, naphthalene, anthracene, fluorene, phenanthrene, pyridine, pyrimidine, triazine, pyrrole, furan, thiophene, quinoline, isoquinoline, indene, benzofuran, benzothiophene, indole, indoline, carbazole, carboline, benzoxazole, benzothiazole, quinoxaline, benzimidazole, pyrazole, dibenzofuran, dibenzothiophene, naphthyridine, phenanthroline, and acridine.

These may have a substituent, and examples of the substituent include the same substituents as those exemplified as the "substituent" in the "silyl group having a substituent", the "phosphino group having a substituent", the "phosphine oxide group having a substituent", or the "aromatic hydrocarbon group having 6 to 25 ring-forming carbon atoms and having a substituent" represented by R₁ to R₄ in the general formula (1). Further, these substituents may bind to each other to form a ring via a single bond, a substituted or unsubstituted methylene group, an oxygen atom, or a sulfur atom.

X₂ in the general formula (2) and the general formula (3) represents B, P, P=O, or P=S. B is defined as a boron atom, P is defined as a phosphorus atom, P=O is defined as a phosphorus atom to which an oxygen atom is bonded via a double bond, and P=S is defined as a phosphorus atom to which a sulfur atom is bonded via a double bond.

Y₁ to Y₃ in the general formula (2) and the general formula (3) may be the same or different, and are any one selected from N-R₈, CR₉R₁₀, O, S, Se, and SiR₁₁R₁₂. N-R₈ is a nitrogen atom having R₈ as a substituent, CR₉R₁₀ is a carbon atom having R₉ and R₁₀ as substituents, O is an oxygen atom, S is a sulfur atom, Se is a selenium atom, and SiR₁₁R₁₂ is a silicon atom having R₁₁ and R₁₂ as substituents.

Here, R₈ to R₁₂ may each bind to the adjacent Q₁, Q₂, or Q₃, that is, to Q₁ when Y₁ is N-R₈, CR₉R₁₀, or SiR₁₁R₁₂, to Q₂ or Q₃ when Y₂ is N-R₈, CR₉R₁₀, or SiR₁₁R₁₂, or to Q₃ when Y₃ is N-R₈, CR₉R₁₀, or SiR₁₁R₁₂ to form a ring via a single bond or a linking group such as a substituted or unsubstituted methylene group, an oxygen atom, a sulfur atom, or a monosubstituted amino group.

Further, R₉ and R₁₀, and R₁₁ and R₁₂ may bind to each other to form a ring via a single bond, a substituted or unsubstituted methylene group, an oxygen atom, a sulfur atom, or a monosubstituted amino group.

The definitions of R₈ to R₁₂ will be further described in detail below.

When Y₁ to Y₃ in the general formula (2) and the general formula (3) are N-R₈, CR₉R₁₀, O, S, Se, or SiR₁₁R₁₂, specific examples of the "linear or branched alkyl group having 1 to 6 carbon atoms", the "cycloalkyl group having 5 to 10 carbon atoms", or the "linear or branched alkenyl group having 2 to 6 carbon atoms" in the "linear or branched alkyl group having 1 to 6 carbon atoms which may have a substituent", the "cycloalkyl group having 5 to 10 carbon atoms which may have a substituent", or the "linear or branched alkenyl group having 2 to 6 carbon atoms which may have a substituent" represented by R₈ to R₁₂ include a methyl group, an ethyl group, an n-propyl group, an isopropyl group, an n-butyl group, an isobutyl group, a tert-butyl group, an n-pentyl group, an isopentyl group, a neopentyl group, an n-hexyl group, a cyclopentyl group, a cyclohexyl group, a 1-adamantyl group, a 2-adamantyl group, a vinyl group, an allyl group, an isopropenyl group, and a 2-butenyl group.

These groups may have a substituent, and examples of the substituent include the same substituents as those exemplified as the "substituent" in the "linear or branched alkyl group having 1 to 6 carbon atoms and having a substituent" represented by R₁ to R₇ in the general formula (1) .

When Y₁ to Y₃ in the general formula (2) and the general formula (3) are N-R₈, CR₉R₁₀, O, S, Se, or SiR₁₁R₁₂, specific examples of the "linear or branched alkyloxy group having 1 to 6 carbon atoms" or the "cycloalkyloxy group having 5 to 10 carbon atoms" in the "linear or branched alkyloxy group having 1 to 6 carbon atoms which may have a substituent" or the "cycloalkyloxy group having 5 to 10 carbon atoms which may have a substituent" represented by R₈ to R₁₂ include a methyloxy group, an ethyloxy group, an n-propyloxy group, an isopropyloxy group, an n-butyloxy group, a tert-butyloxy group, an n-pentyloxy group, an n-hexyloxy group, a cyclopentyloxy group, a cyclohexyloxy group, a cycloheptyloxy group, a cyclooctyloxy group, a 1-adamantyloxy group, and a 2-adamantyloxy group.

These groups may have a substituent, and examples of the substituent include the same substituents as those exemplified as the "substituent" in the "linear or branched alkyl group having 1 to 6 carbon atoms and having a substituent" represented by R₁ to R₇ in the general formula (1) .

When Y₁ to Y₃ in the general formula (2) and the general formula (3) are N-R₈, CR₉R₁₀, O, S, Se, or SiR₁₁R₁₂, specific examples of the "aromatic hydrocarbon group" or the "aromatic heterocyclic group" in the "substituted or unsubstituted aromatic hydrocarbon group" or the "substituted or unsubstituted aromatic heterocyclic group" represented by R₈ to R₁₂ include a phenyl group, a biphenylyl group, a terphenylyl group, a naphthyl group, an anthracenyl group, a phenanthrenyl group, a pyridyl group, a pyrimidinyl group, a triazinyl group, a furyl group, a pyrrolyl group, and a thienyl group.

These groups may have a substituent, and examples of the substituent include the same substituents as those exemplified as the "substituent" in the "silyl group having a substituent", the "phosphino group having a substituent", the "phosphine oxide group having a substituent", or the "aromatic hydrocarbon group having 6 to 25 ring-forming carbon atoms and having a substituent" represented by R₁ to R₄ in the general formula (1).

When Y₁ to Y₃ in the general formula (2) and the general formula (3) are N-R₈, CR₉R₁₀, O, S, Se, or SiR₁₁R₁₂, specific examples of the "aryloxy group" in the "substituted or unsubstituted aryloxy group" represented by R₈ to R₁₂ include a phenyloxy group, a biphenylyloxy group, a terphenylyloxy group, a naphthyloxy group, an anthracenyloxy group, a phenanthrenyloxy group, a fluorenyloxy group, an indenyloxy group, a pyrenyloxy group, and a perylenyloxy group.

These groups may have a substituent, and examples of the substituent include the same substituents as those exemplified as the "substituent" in the "silyl group having a substituent", the "phosphino group having a substituent", the "phosphine oxide group having a substituent", or the "aromatic hydrocarbon group having 6 to 25 ring-forming carbon atoms and having a substituent" represented by R₁ to R₄ in the general formula (1).

In the general formula (2) and the general formula (3), the "aromatic hydrocarbon" or the "aromatic heterocyclic ring" in the "substituted or unsubstituted aromatic hydrocarbon" or the "substituted or unsubstituted aromatic heterocyclic ring" represented by Q₁ to Q₃ are preferably benzene, naphthalene, phenanthrene, pyridine, pyrimidine, indene, benzofuran, benzothiophene, or indole, and more preferably benzene or naphthalene.

In the general formula (2) and the general formula (3), when Y₁ to Y₃ are N-R₈, CR₉R₁₀, or SiR₁₁R₁₂, R₈ to R₁₂ are preferably a linear or branched alkyl group having 1 to 6 carbon atoms which may have a substituent, a cycloalkyl group having 5 to 10 carbon atoms which may have a substituent, a linear or branched alkenyl group having 2 to 6 carbon atoms which may have a substituent, a linear or branched alkyloxy group having 1 to 6 carbon atoms which may have a substituent, a cycloalkyloxy group having 5 to 10 carbon atoms which may have a substituent, a substituted or unsubstituted aromatic hydrocarbon group, or a substituted or unsubstituted aryloxy group, R₈ is more preferably a linear or branched alkyl group having 1 to 6 carbon atoms which may have a substituent, a cycloalkyl group having 5 to 10 carbon atoms which may have a substituent, a linear or branched alkenyl group having 2 to 6 carbon atoms which may have a substituent, or a substituted or unsubstituted aromatic hydrocarbon group.

In the general formula (2) and the general formula (3), Y₁ is preferably N-R₈, O, or S, and more preferably O or S.

Further, in the general formula (2), it is preferred that at least one of Y₂ and Y₃ is N-R₈, and it is more preferred that Y₂ and Y₃ are N-R₈. Here, R₈ is preferably a "substituted or unsubstituted aromatic hydrocarbon group", and more preferably a substituted or unsubstituted phenyl group, a biphenylyl group, a terphenylyl group, or a naphthyl group.

According to the invention, the general formula (2) or the general formula (3) can form a skeleton structure represented by the following general formula (4), general formula (5), general formula (6), or general formula (7).

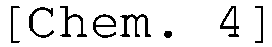

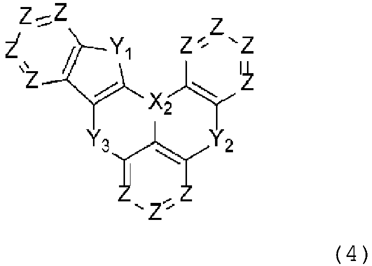

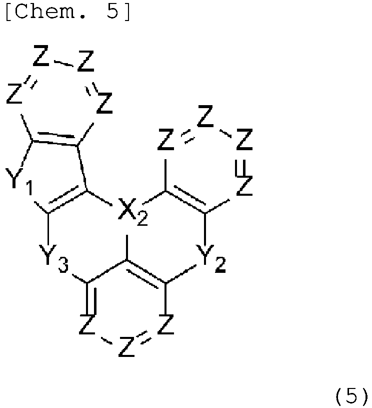

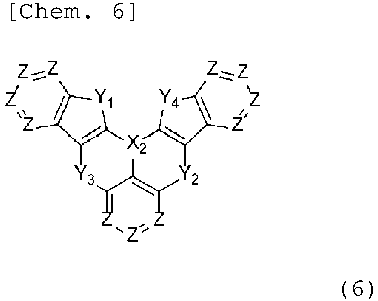

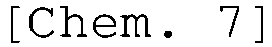

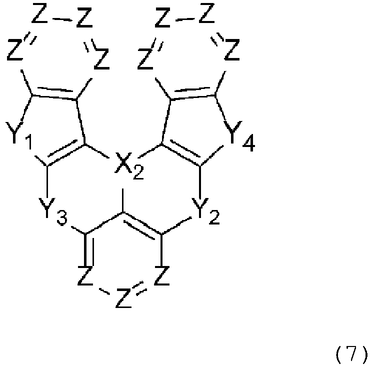

In the general formula (4) to the general formula (7), X₂, Y₁, Y₂, and Y₃ have the same definitions as in the general formula (2) and the general formula (3).

In the general formula (6) to the general formula (7), Y₄ is any one selected from N-R₈, CR₉R₁₀, O, S, Se, or SiR₁₁R₁₂, and R₈ to R₁₂ have the same definitions as in the general formula (2) and the general formula (3).

In the general formula (4) to the general formula (7), Z's may be the same or different, respectively, and are CR₁₃ or N (a nitrogen atom), and R₁₃'s thereof may be the same or different, and represent a hydrogen atom, a deuterium atom, a halogen atom, a cyano group, a nitro group, a linear or branched alkyl group having 1 to 6 carbon atoms which may have a substituent, a cycloalkyl group having 5 to 10 carbon atoms which may have a substituent, a linear or branched alkyloxy group having 1 to 6 carbon atoms which may have a substituent, a linear or branched alkylthioxy group having 1 to 6 carbon atoms which may have a substituent, a linear or branched alkylamino group having 1 to 6 carbon atoms which may have a substituent, a linear or branched alkylsilyl group having 3 to 10 carbon atoms which may have a substituent, a substituted or unsubstituted aromatic hydrocarbon group, a substituted or unsubstituted aromatic heterocyclic group, a substituted or unsubstituted aryloxy group, a substituted or unsubstituted arylthioxy group, a substituted or unsubstituted arylamino group, or a substituted or unsubstituted arylsilyl group. Further, R₁₃'s thereof may bind to each other or each bind to the adjacent substituent to form an alicyclic or aromatic monocyclic or polycyclic ring, and a carbon atom of the alicyclic or aromatic monocyclic or polycyclic ring may be substituted with one or a plurality of heteroatoms selected from N, S, and O.

Specific examples of the "linear or branched alkyl group having 1 to 6 carbon atoms" or the "cycloalkyl group having 5 to 10 carbon atoms" in the "linear or branched alkyl group having 1 to 6 carbon atoms which may have a substituent" or the "cycloalkyl group having 5 to 10 carbon atoms which may have a substituent" represented by R₁₃ in the general formula (4) to the general formula (7) include a methyl group, an ethyl group, an n-propyl group, an isopropyl group, an n-butyl group, an isobutyl group, a tert-butyl group, an n-pentyl group, an isopentyl group, a neopentyl group, an n-hexyl group, a cyclopentyl group, a cyclohexyl group, a 1-adamantyl group, and a 2-adamantyl group.

These groups may have a substituent, and examples of the substituent include the same substituents as those exemplified as the "substituent" in the "linear or branched alkyl group having 1 to 6 carbon atoms and having a substituent" represented by R₁ to R₇ in the general formula (1) .

Specific examples of the "linear or branched alkyloxy group having 1 to 6 carbon atoms" in the "linear or branched alkyloxy group having 1 to 6 carbon atoms which may have a substituent" represented by R₁₃ in the general formula (4) to the general formula (7) include a methyloxy group, an ethyloxy group, an n-propyloxy group, an isopropyloxy group, an n-butyloxy group, a tert-butyloxy group, an n-pentyloxy group, and an n-hexyloxy group.

These groups may have a substituent, and examples of the substituent include the same substituents as those exemplified as the "substituent" in the "linear or branched alkyl group having 1 to 6 carbon atoms and having a substituent" represented by R₁ to R₇ in the general formula (1) .

Specific examples of the "linear or branched alkylthioxy group having 1 to 6 carbon atoms" in the "linear or branched alkylthioxy group having 1 to 6 carbon atoms which may have a substituent" represented by R₁₃ in the general formula (4) to the general formula (7) include a methylthioxy group, an ethylthioxy group, an n-propylthioxy group, an isopropylthioxy group, an n-butylthioxy group, an isobutylthioxy group, a tert-butylthioxy group, an n-pentylthioxy group, an isopentylthioxy group, a neopentylthioxy group, and an n-hexylthioxy group.

These groups may have a substituent, and examples of the substituent include the same substituents as those exemplified as the "substituent" in the "linear or branched alkyl group having 1 to 6 carbon atoms and having a substituent" represented by R₁ to R₇ in the general formula (1) .

Specific examples of the "linear or branched alkylamino group having 1 to 6 carbon atoms" in the "linear or branched alkylamino group having 1 to 6 carbon atoms which may have a substituent" represented by R₁₃ in the general formula (4) to the general formula (7) include a methylamine group, an ethylamine group, an n-propylamine group, an isopropylamine group, an n-butylamine group, an isobutylamine group, a tert-butylamine group, an n-pentylamine group, an isopentylamine group, a neopentylamine group, and an n-hexylamine group.

These groups may have a substituent, and examples of the substituent include the same substituents as those exemplified as the "substituent" in the "linear or branched alkyl group having 1 to 6 carbon atoms and having a substituent" represented by R₁ to R₇ in the general formula (1) .

Specific examples of the "linear or branched alkylsilyl group having 3 to 10 carbon atoms" in the "linear or branched alkylsilyl group having 3 to 10 carbon atoms which may have a substituent" represented by R₁₃ in the general formula (4) to the general formula (7) include a trimethylsilyl group, a triethylsilyl group, a tri-n-propylsilyl group, a triisopropylsilyl group, an n-butyldimethylsilyl group, an isobutyldimethylsilyl group, and a tert-butyldimethylsilyl group.

These groups may have a substituent, and examples of the substituent include the same substituents as those exemplified as the "substituent" in the "linear or branched alkyl group having 1 to 6 carbon atoms and having a substituent" represented by R₁ to R₇ in the general formula (1) .

Specific examples of the "aromatic hydrocarbon group" or the "aromatic heterocyclic group" in the "substituted or unsubstituted aromatic hydrocarbon group", or the "substituted or unsubstituted aromatic heterocyclic group" represented by R₁₃ in the general formula (4) to the general formula (7) include a phenyl group, a biphenylyl group, a terphenylyl group, a naphthyl group, an anthracenyl group, a phenanthrenyl group, a pyridyl group, a pyrimidinyl group, a triazinyl group, a furyl group, a pyrrolyl group, and a thienyl group.

These groups may have a substituent, and examples of the substituent include the same substituents as those exemplified as the "substituent" in the "silyl group having a substituent", the "phosphino group having a substituent", the "phosphine oxide group having a substituent", or the "aromatic hydrocarbon group having 6 to 25 ring-forming carbon atoms and having a substituent" represented by R₁ to R₄ in the general formula (1).

Specific examples of the "aryloxy group" in the "substituted or unsubstituted aryloxy group" represented by R₁₃ in the general formula (4) to the general formula (7) include a phenyloxy group, a biphenylyloxy group, a terphenylyloxy group, a naphthyloxy group, an anthracenyloxy group, a phenanthrenyloxy group, a fluorenyloxy group, an indenyloxy group, a pyrenyloxy group, and a perylenyloxy group.

These groups may have a substituent, and examples of the substituent include the same substituents as those exemplified as the "substituent" in the "silyl group having a substituent", the "phosphino group having a substituent", the "phosphine oxide group having a substituent", or the "aromatic hydrocarbon group having 6 to 25 ring-forming carbon atoms and having a substituent" represented by R₁ to R₄ in the general formula (1).

Specific examples of the "arylthioxy group" in the "substituted or unsubstituted arylthioxy group" represented by R₁₃ in the general formula (4) to the general formula (7) include a phenylthioxy group, a biphenylylthioxy group, a terphenylylthioxy group, a naphthylthioxy group, an anthracenylthioxy group, a phenanthrenylthioxy group, a fluorenylthioxy group, an indenylthioxy group, a pyrenylthioxy group, and a perylenylthioxy group.

These groups may have a substituent, and examples of the substituent include the same substituents as those exemplified as the "substituent" in the "silyl group having a substituent", the "phosphino group having a substituent", the "phosphine oxide group having a substituent", or the "aromatic hydrocarbon group having 6 to 25 ring-forming carbon atoms and having a substituent" represented by R₁ to R₄ in the general formula (1).

Specific examples of the "arylamino group" in the "substituted or unsubstituted arylamino group" represented by R₁₃ in the general formula (4) to the general formula (7) include a phenylamino group, a biphenylylamino group, a terphenylylamino group, a naphthylamino group, an anthracenylamino group, a phenanthrenylamino group, a fluorenylamino group, an indenylamino group, a pyrenylamino group, and a perylenylamino group.

These groups may have a substituent, and examples of the substituent include the same substituents as those exemplified as the "substituent" in the "silyl group having a substituent", the "phosphino group having a substituent", the "phosphine oxide group having a substituent", or the "aromatic hydrocarbon group having 6 to 25 ring-forming carbon atoms and having a substituent" represented by R₁ to R₄ in the general formula (1).

Specific examples of the "arylsilyl group" in the "substituted or unsubstituted arylsilyl group" represented by R₁₃ in the general formula (4) to the general formula (7) include a triphenylsilyl group, a trinaphthylsilyl group, and a terphenylylsilyl group.

These groups may have a substituent, and examples of the substituent include the same substituents as those exemplified as the "substituent" in the "silyl group having a substituent", the "phosphino group having a substituent", the "phosphine oxide group having a substituent", or the "aromatic hydrocarbon group having 6 to 25 ring-forming carbon atoms and having a substituent" represented by R₁ to R₄ in the general formula (1).

### Advantageous Effects of Invention

The arylamine compound represented by the general formula (1) according to the invention has larger hole mobility, a superior electron blocking ability, and superior amorphousness, and is in a more stable thin film state compared to conventional hole transport materials, and therefore, the organic EL device of the invention using such a compound as a constituent material of the hole transport layer can achieve an organic EL device having high efficiency, a low driving voltage, and a long lifetime.

Further, in the invention, the hole transport layer is configured to have a two-layer structure including a first hole transport layer and a second hole transport layer, and by forming the second hole transport layer located on the light emitting layer side from the arylamine compound of the general formula (1), the electron blocking performance of the arylamine compound can be fully utilized, and thus, an organic EL device having higher efficiency and a longer lifetime can be achieved.

### Brief Description of Drawings

[FIG. 1] FIG. 1 is a drawing showing the structural formulae of compounds (1-1) to (1-15) as examples of an arylamine compound represented by the general formula (1).
[FIG. 2] FIG. 2 is a drawing showing the structural formulae of compounds (1-16) to (1-27) as examples of an arylamine compound represented by the general formula (1).
[FIG. 3] FIG. 3 is a drawing showing the structural formulae of compounds (1-28) to (1-39) as examples of an arylamine compound represented by the general formula (1).
[FIG. 4] FIG. 4 is a drawing showing the structural formulae of compounds (1-40) to (1-51) as examples of an arylamine compound represented by the general formula (1).
[FIG. 5] FIG. 5 is a drawing showing the structural formulae of compounds (1-52) to (1-63) as examples of an arylamine compound represented by the general formula (1).
[FIG. 6] FIG. 6 is a drawing showing the structural formulae of compounds (1-64) to (1-76) as examples of an arylamine compound represented by the general formula (1).
[FIG. 7] FIG. 7 is a drawing showing the structural formulae of compounds (2-1) to (2-11) as examples of a compound represented by the general formula (2).
[FIG. 8] FIG. 8 is a drawing showing the structural formulae of compounds (2-12) to (2-26) as examples of a compound represented by the general formula (2).
[FIG. 9] FIG. 9 is a drawing showing the structural formulae of compounds (3-1) to (3-12) as examples of a compound represented by the general formula (3).
[FIG. 10] FIG. 10 is a diagram illustrating a configuration of an organic EL device of Examples and Comparative Examples of the invention.

### Description of Embodiments

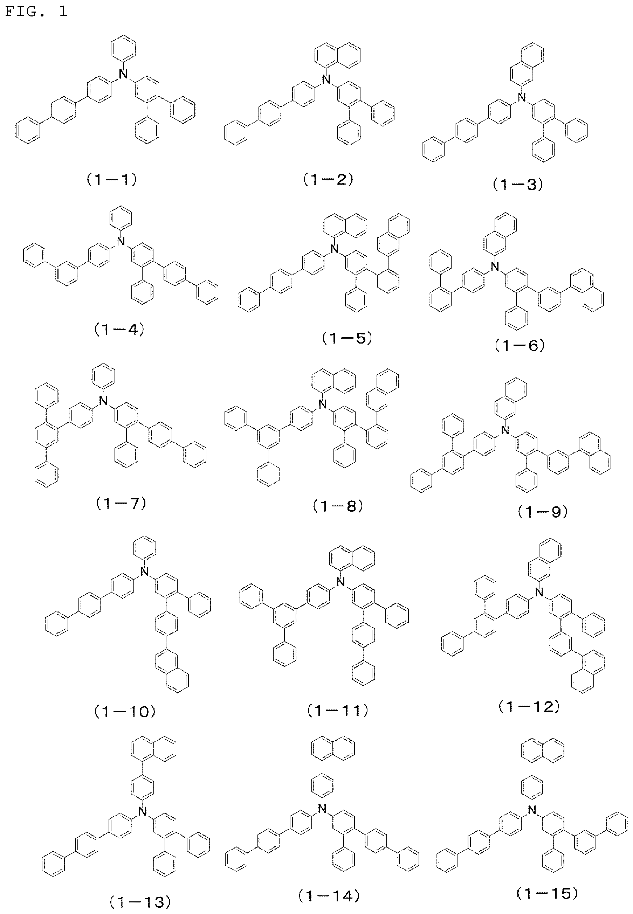

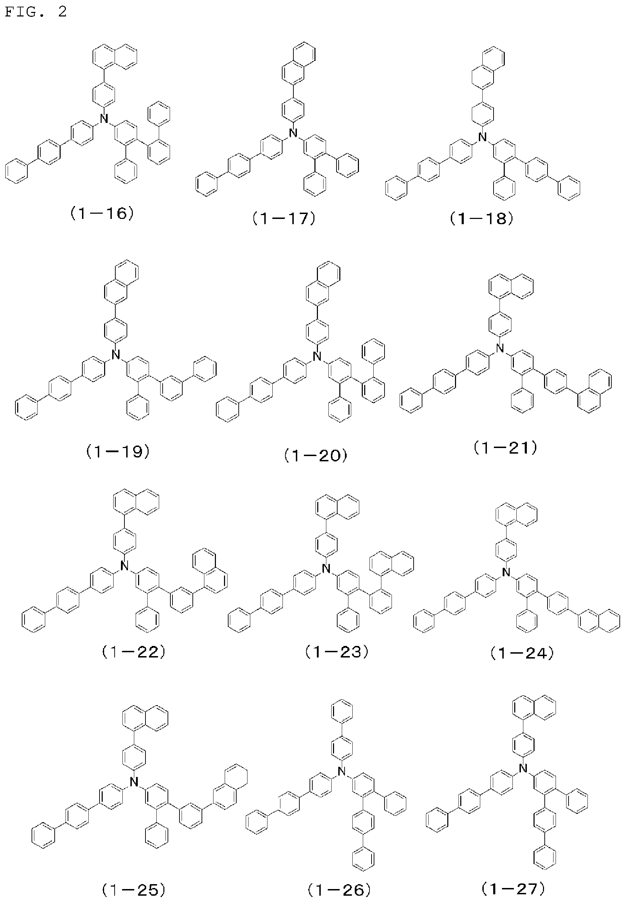

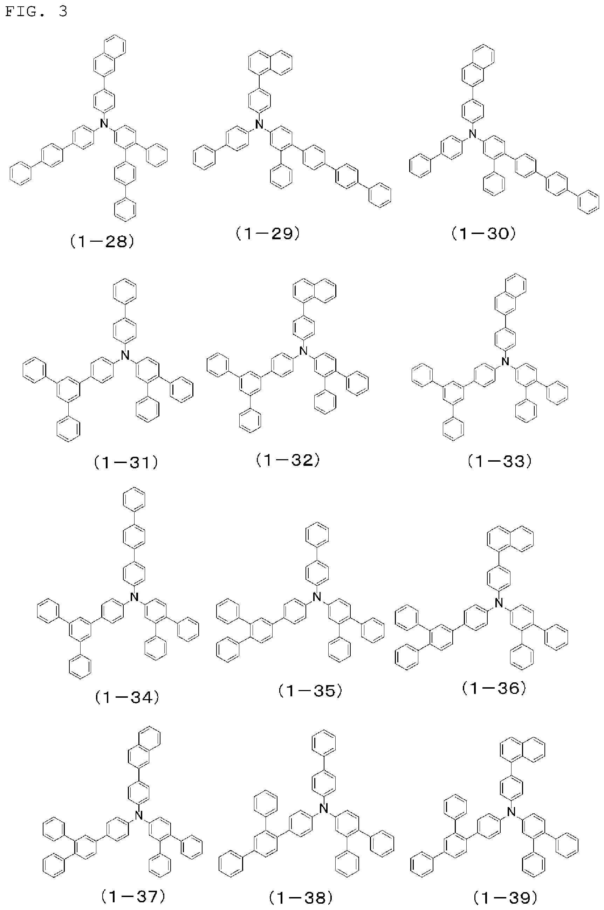

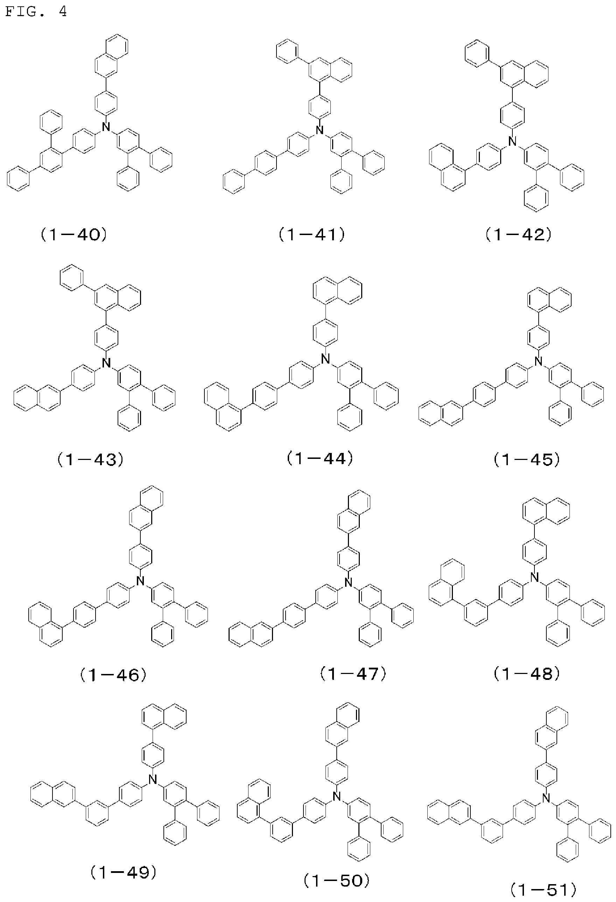

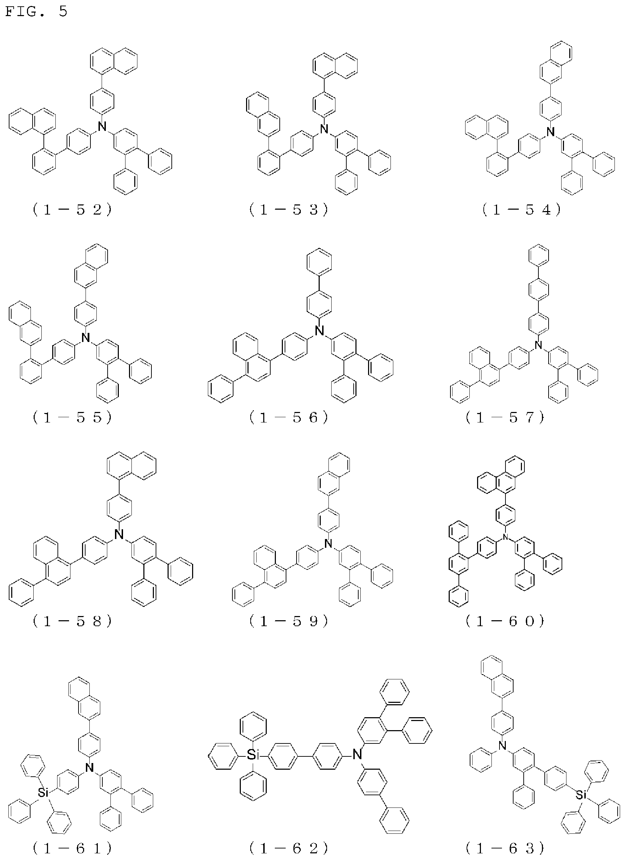

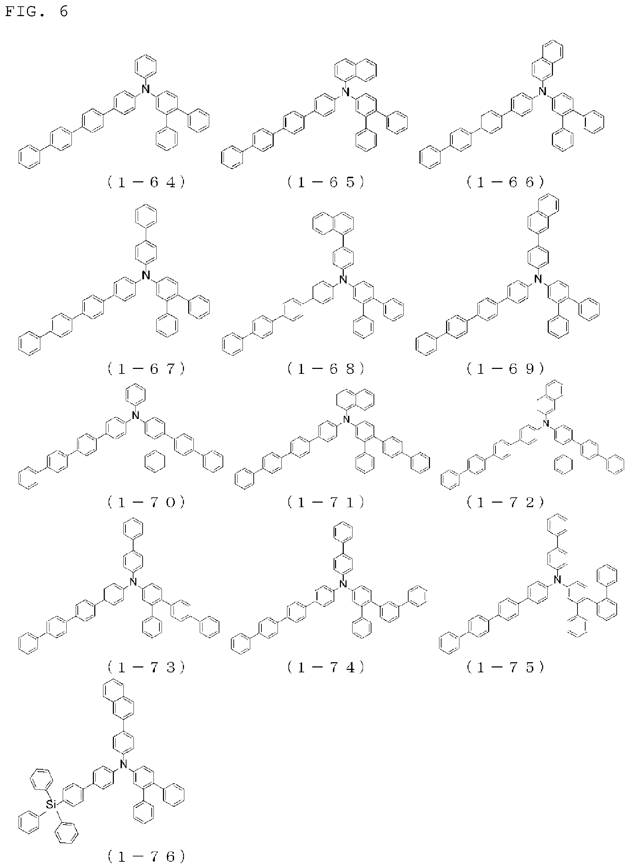

Specific examples of preferred compounds among the arylamine compounds represented by the general formula (1) preferably used in the organic EL device of the invention are shown in FIGS. 1 to 6, but the invention is not limited to these compounds.

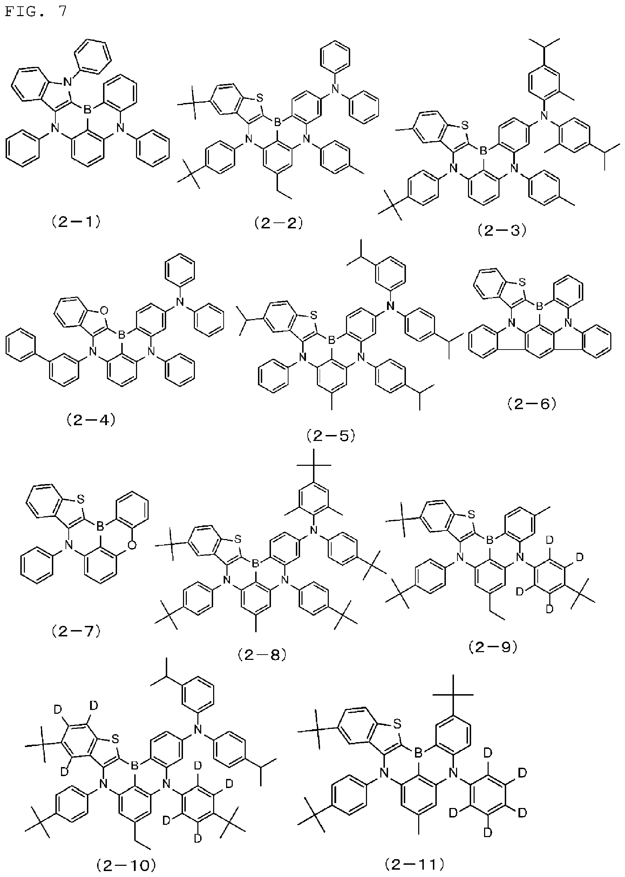

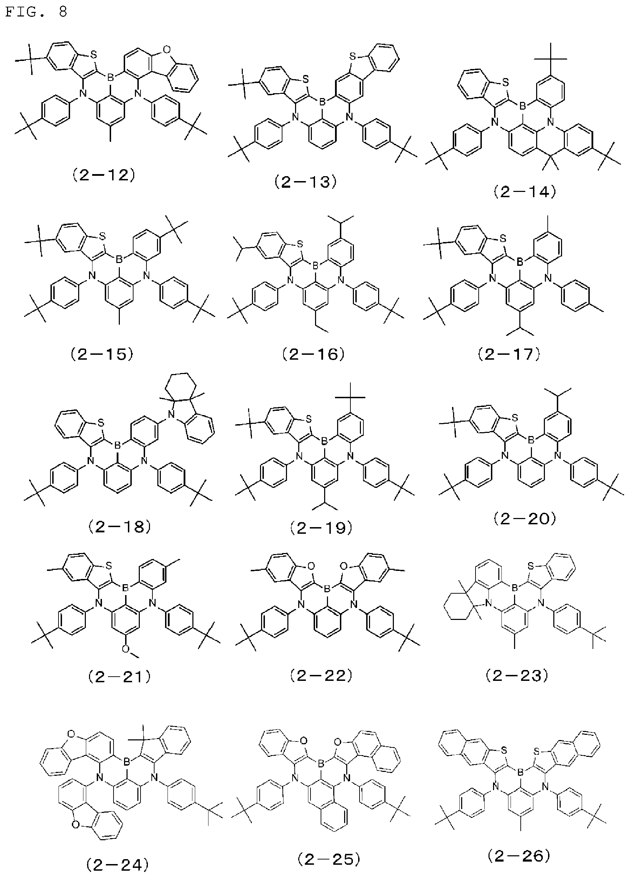

Specific examples of preferred compounds among the compounds represented by the general formula (2) preferably used in the organic EL device of the invention are shown in FIGS. 7 to 8, but the invention is not limited to these compounds.

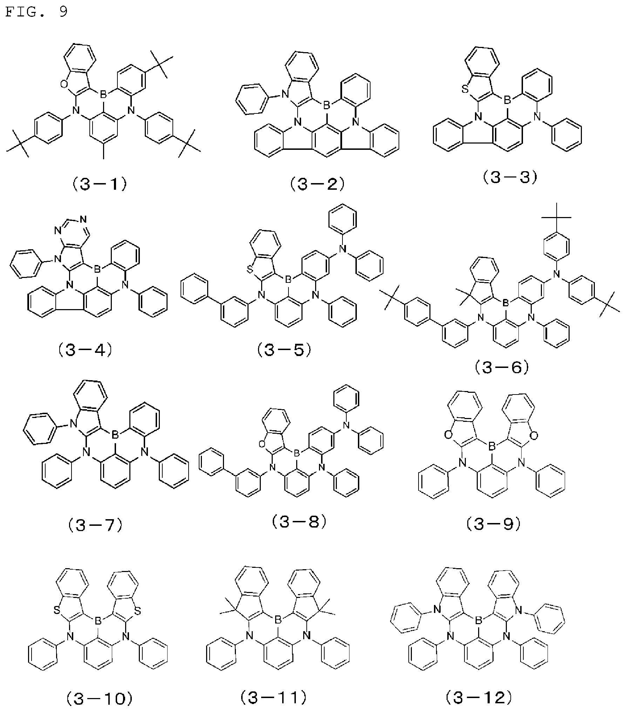

Specific examples of preferred compounds among the compounds represented by the general formula (3) preferably used in the organic EL device of the invention are shown in FIG. 9, but the invention is not limited to these compounds.

The arylamine compound represented by the general formula (1) was purified by purification using a column chromatograph, adsorption purification using silica gel, activated carbon, activated clay, or the like, a recrystallization or crystallization method using a solvent, a sublimation purification method, or the like. The compound was identified by an NMR analysis. A glass transition point (Tg) and a work function were measured as physical property values. The glass transition point (Tg) can serve as an index of stability in a thin film state, and the work function can serve as an index of hole transportability and hole blocking performance. In addition, as the compound used in the organic EL device of the invention, a compound finally purified by a sublimation purification method after performing purification using a column chromatograph, adsorption purification using silica gel, activated carbon, activated clay, or the like, or purification through a recrystallization or crystallization method using a solvent, or the like was used.

The glass transition point (Tg) was measured by a high-sensitive differential scanning calorimeter (DSC3100SA manufactured by Bruker AXS) using a powder.

The work function was determined using an ionization potential measuring device (PYS-202 manufactured by Sumitomo Heavy Industries, Ltd.) by fabricating a 100 nm-thick thin film on an ITO substrate.

As the structure of the organic EL device of the invention, a structure including an anode, a hole transport layer, a light emitting layer, an electron transport layer, and a cathode sequentially on a substrate, a structure including a hole injection layer between the anode and the hole transport layer, a structure including a hole blocking layer between the light emitting layer and the electron transport layer, and a structure including an electron injection layer between the electron transport layer and the cathode can be exemplified. Some of the organic layers in the multilayer structure may be omitted or combined, and for example, a configuration in which the hole injection layer and the hole transport layer are combined, a configuration in which the electron injection layer and the electron transport layer are combined, and so on can be also adopted. Further, a configuration in which two or more organic layers having the same function are laminated can be adopted, and a configuration in which two hole transport layers are laminated, a configuration in which two light emitting layers are laminated, a configuration in which two electron transport layers are laminated, and so on can be also adopted. The structure of the organic EL device of the invention is preferably such that the hole transport layer has a two-layer structure including a first hole transport layer and a second hole transport layer, and the second hole transport layer in this case is preferably adjacent to a light emitting layer, and in such a case, it can function as an electron blocking layer.

As the anode of the organic EL device of the invention, an electrode material having a high work function such as ITO or gold is used. As the hole injection layer of the organic EL device of the invention, a material such as a starburst-type triphenylamine derivative or various triphenylamine tetramers; a porphyrin compound represented by copper phthalocyanine; an acceptor-type heterocyclic compound such as hexacyanoazatriphenylene or a coating-type polymer material, or the like can be used. These materials can be formed into a thin film by a known method such as a spin coating method or an inkjet method in addition to a vapor deposition method.

As the hole transporting material that can be used as the hole transport layer of the organic EL device of the invention, it is preferred to use a benzidine derivative such as N,N'-diphenyl-N,N'-di(m-tolyl)benzidine (TPD), NPD, or N,N,N',N'-tetrabiphenylylbenzidine, 1,1-bis[4-(di-4-tolylamino)phenyl]cyclohexane (TAPC), particularly, an arylamine compound having a structure in which two triphenylamine structures are joined within a molecule via a single bond or a divalent group which does not contain a heteroatom, for example, N,N,N',N'-tetrabiphenylylbenzidine or the like, or an arylamine compound having only one triphenylamine structure within a molecule such as a triphenylamine derivative represented by the general formula (1). Further, it is possible to use arylamine compounds having a structure in which three or more triphenylamine structures are joined within a molecule via a single bond or a divalent group which does not contain a heteroatom, for example, organic amine compounds such as various triphenylamine derivatives including various triphenylamine trimers and tetramers, etc. These may be individually deposited into a film, but may be used as a single layer deposited by being mixed with another material, or may be formed into a laminate structure of individually deposited layers, mixedly deposited layers, or an individually deposited layer and a mixedly deposited layer. These materials can be formed into a thin film by a known method such as a spin coating method or an inkjet method in addition to a vapor deposition method.

Further, in the hole injection layer or the hole transport layer, a material obtained by further P-doping a material which is commonly used for the layer with trisbromophenylamine hexachloroantimony, a radialene derivative (see, for example, PTL 6), or the like, or a polymer compound having a structure of a benzidine derivative such as TPD in its partial structure, or the like can be used.

As the second hole transport layer located on the light emitting layer side of the organic EL device of the invention, the arylamine compound represented by the general formula (1) is used. Examples of a hole transporting material that can be mixed or can be used at the same time with the arylamine compound represented by the general formula (1) include compounds having an electron blocking effect such as carbazole derivatives including 4,4',4"-tri(N-carbazolyl)triphenylamine (TCTA), 9,9-bis[4-(carbazol-9-yl)phenyl]fluorene, 1,3-bis(carbazol-9-yl)benzene (mCP), and 2,2-bis(4-carbazol-9-ylphenyl)adamantane (Ad-Cz), and compounds having a triphenylsilyl group and a triarylamine structure represented by 9-[4-(carbazol-9-yl)phenyl]-9-[4-(triphenylsilyl)phenyl]-9H-fluorene.

These may be individually deposited into a film, but may be used as a single layer deposited by being mixed with another material, or may be formed into a laminate structure of individually deposited layers, mixedly deposited layers, or an individually deposited layer and a mixedly deposited layer. These materials can be formed into a thin film by a known method such as a spin coating method or an inkjet method in addition to a vapor deposition method.

As the light emitting layer of the organic EL device of the invention, a blue light emitting dopant such as a pyrene derivative having a pyrene skeleton in the molecule, or a compound represented by the general formula (2) or the general formula (3) is preferably used. Furthermore, in addition to a metal complex of a quinolinol derivative such as Alq₃, various metal complexes, an anthracene derivative, a bis(styryl)benzene derivative, a pyrene derivative, an oxazole derivative, a polyparaphenylene vinylene derivative, or the like can be used. Further, the light emitting layer may be made of a host material and a dopant material. In that case, as the host material, an anthracene derivative having an anthracene skeleton in the molecule is preferably used. Furthermore, in addition to the light emitting materials, a heterocyclic compound having an indole ring as a partial structure of a fused ring, a heterocyclic compound having a carbazole ring as a partial structure of a fused ring, a carbazole derivative, a thiazole derivative, a benzimidazole derivative, a polydialkylfluorene derivative, or the like can be used. Further, as the dopant material, a pyrene derivative having a pyrene skeleton in the molecule, or a compound represented by the general formula (2) or the general formula (3) is preferably used, but in addition thereto, a heterocyclic compound having an indole ring as a partial structure of a fused ring, a heterocyclic compound having a carbazole ring as a partial structure of a fused ring, a carbazole derivative, a thiazole derivative, a benzimidazole derivative, a polydialkylfluorene derivative, quinacridone, coumarin, rubrene, perylene, derivatives thereof, a benzopyran derivative, an indenophenanthrene derivative, a rhodamine derivative, an aminostyryl derivative, or the like can be used. These may be individually deposited into a film, but may be used as a single layer deposited by being mixed with another material, or may be formed into a laminate structure of individually deposited layers, mixedly deposited layers, or an individually deposited layer and a mixedly deposited layer.

Further, as the light emitting material, a phosphorescent material can be also used. As the phosphorescent material, a phosphorescent material of a metal complex such as iridium or platinum can be used. A blue phosphorescent material such as FIrpic or FIr6 is used, and as the host material at that time, a carbazole derivative such as 4,4'-di(N-carbazolyl)biphenyl (CBP), TCTA, or mCP, or the like can be used as a hole injecting and transporting host material. As an electron transporting host material, p-bis(triphenylsilyl)benzene (UGH2), 2,2',2"-(1,3,5-phenylene)-tris(1-phenyl-1H-benzimidazole) (TPBI), or the like can be used, and a high-performance organic EL device can be fabricated.

In order to avoid concentration quenching, the doping of the host material with the phosphorescent light emitting material is preferably performed by co-deposition in a range of 1 to 30 wt% with respect to the whole light emitting layer.

Further, as the light emitting material, it is also possible to use a material that emits delayed fluorescence such as a CDCB derivative including PIC-TRZ, CC2TA, PXZ-TRZ, 4CzIPN, and the like (see, for example, NPL 3).

These materials can be formed into a thin film by a known method such as a spin coating method or an inkjet method in addition to a vapor deposition method.

As the hole blocking layer of the organic EL device of the invention, a compound having a hole blocking effect such as various rare earth complexes, a triazole derivative, a triazine derivative, or an oxadiazole derivative in addition to a phenanthroline derivative such as bathocuproine (BCP) or a metal complex of a quinolinol derivative such as aluminum(III) bis(2-methyl-8-quinolinate)-4-phenylphenolate (BAlq) can be used. These materials may also serve as the material of the electron transport layer. These may be individually deposited into a film, but may be used as a single layer deposited by being mixed with another material, or may be formed into a laminate structure of individually deposited layers, mixedly deposited layers, or an individually deposited layer and a mixedly deposited layer. These materials can be formed into a thin film by a known method such as a spin coating method or an inkjet method in addition to a vapor deposition method.

As the electron transport layer of the organic EL device of the invention, a metal complex of a quinolinol derivative such as Alq₃ or BAlq, various metal complexes, a triazole derivative, a triazine derivative, an oxadiazole derivative, a pyridine derivative, a pyrimidine derivative, a benzimidazole derivative, a thiadiazole derivative, an anthracene derivative, a carbodiimide derivative, a quinoxaline derivative, a pyridoindole derivative, a phenanthroline derivative, a silole derivative, or the like can be used. These may be individually deposited into a film, but may be used as a single layer deposited by being mixed with another material, or may be formed into a laminate structure of individually deposited layers, mixedly deposited layers, or an individually deposited layer and a mixedly deposited layer. These materials can be formed into a thin film by a known method such as a spin coating method or an inkjet method in addition to a vapor deposition method.

As the electron injection layer of the organic EL device of the invention, an alkali metal salt such as lithium fluoride or cesium fluoride, an alkaline earth metal salt such as magnesium fluoride, a metal complex of a quinolinol derivative such as lithium quinolinol, a metal oxide such as aluminum oxide, or a metal such as ytterbium (Yb), samarium (Sm), calcium (Ca), strontium (Sr), or cesium (Cs), or the like can be used. However, the electron injection layer may be omitted in the preferred selection of the electron transport layer and the cathode.

Further, in the electron injection layer or the electron transport layer, a material obtained by further N-doping a material which is commonly used for the layer with a metal such as cesium can be used.

As the cathode of the organic EL device of the invention, an electrode material having a low work function such as aluminum, or an alloy having a lower work function such as a magnesium-silver alloy, a magnesium-indium alloy, or an aluminum-magnesium alloy is used as the electrode material.

As a capping layer of the organic EL device of the invention, it is preferred to use an arylamine compound having a structure in which 2 to 6 triphenylamine structures are joined within a molecule via a single bond or a divalent group which does not contain a heteroatom, an amine compound having a benzoazole ring structure, an amine compound having an aromatic heterocyclic group in the molecule, or the like. These may be individually deposited into a film, but may be used as a single layer deposited by mixing different materials, or may be formed into a laminate structure of individually deposited layers, mixedly deposited layers, or an individually deposited layer and a mixedly deposited layer. These materials can be formed into a thin film by a known method such as a spin coating method or an inkjet method in addition to a vapor deposition method.

Hereinafter, embodiments of the invention will be more specifically described with reference to Examples. The invention, however, is not limited to the following Examples.

### Example 1

### <Synthesis of ([1,1':4',1"]terphenyl-4-yl)-(4-naphthalen-1-yl-phenyl)-[1,1':2',1"]terphenyl-4'-yl-amine (1-13)>

To a reaction vessel purged with nitrogen, 33.8 g of [1,1'2',1"]terphenyl-4'-yl-amine, 30.0 g of 1-(4-bromophenyl) naphthalene, 12.2 g of sodium tert-butoxide, and 300 mL of toluene were added, and the mixture was aerated with nitrogen gas under ultrasonic irradiation for 30 minutes. To the vessel, 1.9 g of tris(dibenzylideneacetone)dipalladium(0) and 2.6 g of 2,2'-bis(diphenylphosphino)-1,1'-binaphthyl were added, and the mixture was heated and stirred under reflux for 14 hours. The mixture was cooled to 80°C, and the inorganic substances were removed by hot filtration, and then, the filtrate was concentrated. The residue was recrystallized using toluene and heptane, whereby 37.0 g of a brown solid of (4-naphthalen-1-yl-phenyl)-[1,1':2',1"]terphenyl-4'-yl-amine (yield: 78.1%) was obtained.

To a reaction vessel purged with nitrogen, 8.0 g of the obtained (4-naphthalen-1-yl-phenyl)-[1,1'2',1"]terphenyl-4'-yl-amine was added, and subsequently, 5.0 g of 4-bromo-[1,1':4',1"]terphenyl, 2.3 g of sodium tert-butoxide, and 50 mL of toluene were added, and the mixture was aerated with nitrogen gas under ultrasonic irradiation for 30 minutes. To the vessel, 0.07 g of palladium acetate and 0.30 g of a 50% toluene solution of tri(tert-butylphosphine) were added, and the mixture was heated and stirred under reflux for 4 hours. The mixture was cooled to 80°C, and the inorganic substances were removed by hot filtration, and then, the filtrate was concentrated. The residue was recrystallized using toluene and acetone, whereby 13.8 g of a white solid of ([1,1':4',1"Jterphenyl-4-yl)-(4-naphthalen-1-yl-phenyl)-[1,1':2',1"]terphenyl-4'-yl-amine (1-13) (yield: 78.8%) was obtained.

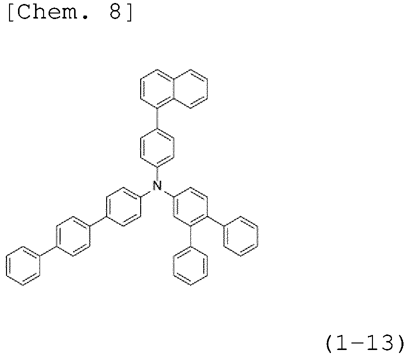

The structure of the obtained white solid was identified using NMR.

The following 37 hydrogen signals were detected by ¹H-NMR (CDCl₃).
δ (ppm) = 8.03-8.06 (1H), 7.82-7.90 (2H), 7.58-7.69 (8H), 7.42-7.53 (8H), 7.13-7.27 (18H).

### Example 2

### <Synthesis of ([1,1':4',1"]terphenyl-4-yl)-(4-naphthalen-2-yl-phenyl)-[1,1':2',1"]terphenyl-4'-yl-amine (1-17)>

The same operation as in Example 1 was performed except that 1-(4-bromophenyl)naphthalene was replaced with 2-(4-bromophenyl)naphthalene, whereby 5.6 g of a light yellow solid of ([1,1':4',1"]terphenyl-4-yl)-(4-naphthalen-2-yl-phenyl)-[1,1':2',1"]terphenyl-4'-yl-amine (1-17) (yield: 57%) was obtained.

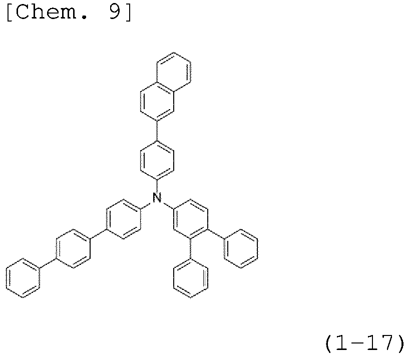

The structure of the obtained light yellow solid was identified using NMR.

The following 37 hydrogen signals were detected by ¹H-NMR (CDCl₃).
δ (ppm) = 7.82-8.05 (3H), 7.52-7.70 (8H), 7.39-7.51 (8H), 7.10-7.29 (18H).

### Example 3

### <Synthesis of (biphenyl-4-yl)-(4-naphthalen-2-yl-phenyl)-[1,1':2',1":4",1‴:4‴:1ʺʺ]-quinquephenyl-5'-yl-amine (1-30) >

To a reaction vessel, 8.0 g of (6-bromo-biphenyl-3-yl)-(4-naphthalen-2-yl-phenyl)-biphenyl-4-yl-amine, 5.7 g of 2-([1,1':4,1"]terphenyl-4-yl)-4,4,5,5-tetramethyl-1,3,2-dioxaborolane, 1.7 g of sodium bicarbonate, 100 mL of tetrahydrofuran, and 30 mL of water were added, and the mixture was aerated with nitrogen gas under ultrasonic irradiation for 30 minutes. To the vessel, 0.2 g of diphenylphosphino ferrocene palladium dichloride was added, and the mixture was stirred under reflux for 12 hours. After the mixture was cooled, 200 mL of methanol was added to the reaction solution, and the mixture was stirred for 1 hour, and then, the precipitated solid was collected by filtration. To the obtained solid, 100 mL of toluene was added, and the mixture was heated to 80°C, and adsorption purification using silica gel and activated clay was performed. The solid was removed by filtration, and the filtrate was concentrated, and then, the residue was recrystallized using toluene and acetone, whereby 8.4 g of a white solid of (biphenyl-4-yl)-(4-naphthalen-2-yl-phenyl)-[1,1':2',1":4",1‴:4‴:1ʺʺ]-quinquephenyl-5'-yl-amine (1-30) (yield: 84%) was obtained.

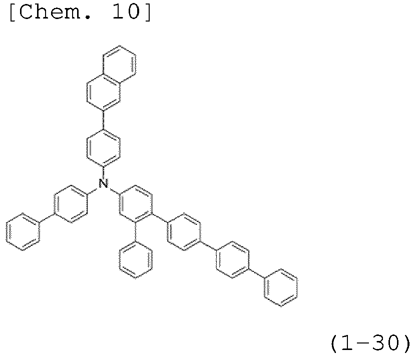

The structure of the obtained white solid was identified using NMR.

The following 41 hydrogen signals were detected by ¹H-NMR (CDCl₃).
δ (ppm) = 8.03 (1H), 7.83-7.90 (3H), 7.73-7.76 (1H), 7.29-7.67 (28H), 7.15-7.24 (8H).

### Example 4

<Synthesis of (4-naphthalen-1-yl-phenyl)-(5'-phenyl-[1,1':3',1"]-terphenyl-4-yl)-[1,1':2',1"]terphenyl-4'-yl-amine (1-32)>

The same operation as in Example 1 was performed except that 4-bromo-[1,1':4',1"]terphenyl was replaced with 4-chloro-5'-phenyl-[1,1':3',1"]terphenyl, whereby 3.7 g of a white solid of (4-naphthalen-1-yl-phenyl)-(5'-phenyl-[1,1':3',1"]terphenyl-4-yl)-[1,1':2',1"]terphenyl-4'-yl-amine (1-32) (yield: 37%) was obtained.

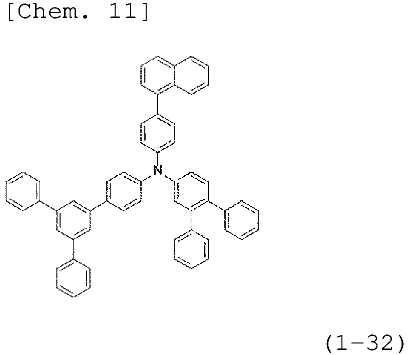

The structure of the obtained white solid was identified using NMR.

The following 41 hydrogen signals were detected by ¹H-NMR (CDCl₃).
δ (ppm) = 8.04-8.06 (1H), 7.64-7.91 (11H), 7.32-7.53 (18H), 7.12-7.29 (11H).

### Example 5

### <Synthesis of ([1,1':4',1"]terphenyl-4-yl)-{4-(3-phenylnaphthalen-1-yl)phenyl}-[1,1':2',1"]terphenyl-4'-yl-amine (1-41)>

The same operation as in Example 1 was performed except that 1-(4-bromophenyl)naphthalene was replaced with 1-(4-chrorophenyl)-3-phenylnaphthalene, whereby 5.0 g of a white solid of ([1,1':4',1"]terphenyl-4-yl)-{4-(3-phenylnaphthalen-1-yl)phenyl}-[1,1':2',1"]terphenyl-4'-yl-amine (1-41) (yield: 47%) was obtained.

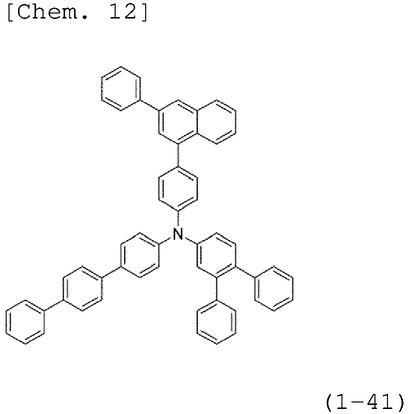

The structure of the obtained white solid was identified using NMR.

The following 41 hydrogen signals were detected by ¹H-NMR (CDCl₃).
δ (ppm) = 8.04-8.05 (2H), 7.94-7.96 (1H), 7.76-7.78 (3H), 7.59-7.67 (8H), 7.43-7. 52 (8H), 7.12-7.22 (19H).

### Example 6

### <Synthesis of (4'-naphthalen-1-yl-biphenyl-4-yl)-(4-naphthalen-1-yl-phenyl)-[1,1':2',1"]terphenyl-4'-yl-amine (1-44)>

The same operation as in Example 1 was performed except that 4-bromo-[1,1':4',1"]terphenyl was replaced with 1-(4'-bromo-biphenyl-4-yl)naphthalene, whereby 4.5 g of a white solid of (4'-naphthalen-1-yl-biphenyl-4-yl)-(4-naphthalen-1-yl-phenyl)-[1,1':2',1"]terphenyl-4'-yl-amine (1-44) (yield: 48%) was obtained.

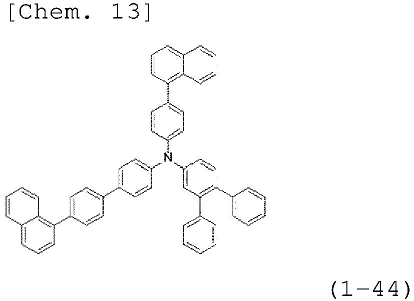

The structure of the obtained white solid was identified using NMR.

The following 39 hydrogen signals were detected by ¹H-NMR (CDCl₃).
δ (ppm) = 8.05-8.07 (1H), 7.98-8.00 (1H), 7.83-7.91 (4H), 7.71-7.73 (2H), 7.63-7. 65 (2H), 7.33-7.57 (19H), 7.12-7.21 (10H).

### Example 7

### <Synthesis of (4-naphthalen-1-yl-phenyl)-{4-(4-phenylnaphthalen-1-yl)phenyl}-[1,1':2',1"]terphenyl-4'-yl-amine (1-58)>

The same operation as in Example 1 was performed except that 4-bromo-[1,1':4',1"]terphenyl was replaced with 1-(4-chlorophenyl)-4-phenylnaphthalene, whereby 4.2 g of a white solid of (4-naphthalen-1-yl-phenyl)-{4-(4-phenylnaphthalen-1-yl)phenyl}-[1,1':2',1"]terphenyl-4'-yl-amine (1-58) (yield: 57%) was obtained.

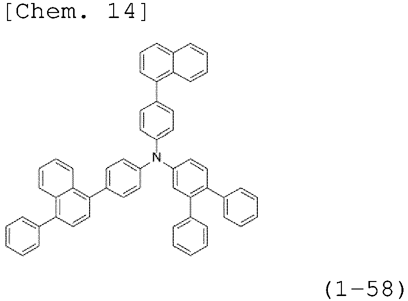

The structure of the obtained white solid was identified using NMR.

The following 39 hydrogen signals were detected by ¹H-NMR (CDCl₃).
δ (ppm) = 8.12-8.14 (1H), 8.05-8.07 (1H), 7.96-7.98 (1H), 7.89-7.91 (1H), 7.83-7. 85 (1H), 7.32-7.55 (24H), 7.14-7.23 (10H).

### Example 8

### <Synthesis of ([1,1':4',1"]terphenyl-4-yl)-(4-naphthalen-1-yl-phenyl)-[1,1':2',1":4",1‴]quaterphenyl-5'-yl-amine (1-14)>

To a reaction vessel, 12.5 g of (4-naphthalen-1-yl-phenyl)-(6-bromo-biphenyl-3-yl)amine, 6.6 g of 4-biphenylboronic acid, 3.5 g of sodium bicarbonate, 100 mL of tetrahydrofuran, and 50 mL of water were added. To the vessel, 0.5 g of diphenylphosphino ferrocene palladium dichloride was added, and the mixture was stirred under reflux for 12 hours. After the mixture was cooled, extraction was performed using ethyl acetate, and the organic layer was concentrated. To the residue, 150 mL of toluene was added, and the mixture was stirred while heating. Thereafter, 8 g of silica gel was added thereto at 80°C, and the mixture was stirred for 1 hour, and then, the solid was removed by hot filtration. The filtrate was concentrated, and the residue was repeatedly recrystallized twice using dichloromethane and heptane, whereby 14.2 g of a white solid of (4-naphthalen-1-yl-phenyl)-[1,1':2',1":4",1‴]quaterphenyl-5'-yl-amine (yield: 97.9%) was obtained.

To a reaction vessel purged with nitrogen, 14.2 g of the obtained (4-naphthalen-1-yl-phenyl)-[1,1':2',1":4",1‴]quaterphenyl-5'-yl-amine was added, and subsequently, 9.2 g of 4-bromo-[1,1' 4',1"]terphenyl, 3.9 g of sodium tert-butoxide, and 140 mL of toluene were added. To the vessel, 0.12 g of palladium acetate and 0.50 g of a 50% toluene solution of tri(tert-butylphosphine) were added, and the mixture was heated and stirred under reflux for 4 hours. After the mixture was cooled to room temperature, 140 mL of methanol was added to the reaction solution, and the mixture was stirred for 1 hour, and then, the precipitated solid was collected by filtration. To the solid, 200 mL of toluene was added, and the mixture was heated to 80°C, and thereafter, 10 g of activated clay and 10 g of silica gel were added thereto. Then, the mixture was stirred for 1 hour, and the solid was removed by hot filtration. The filtrate was concentrated, and the residue was recrystallized using toluene and acetone, whereby 16.9 g of a white solid of ([1,1':4',1"]terphenyl-4-yl)-(4-naphthalen-1-yl-phenyl)-[1,1':2',1":4",1‴]quaterphenyl-5'-yl-amine (1-14) (yield: 82.8%) was obtained.

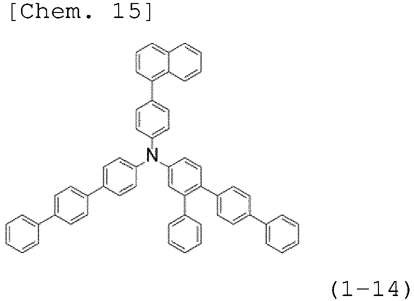

The structure of the obtained white solid was identified using NMR.

The following 41 hydrogen signals were detected by ¹H-NMR (CDCl₃).
δ (ppm) = 8.03-8.06 (1H), 7.89-7.91 (1H), 7.83-7.85 (1H), 7.57-7.67 (10H), 7.27-7.52 (21H), 7.16-7.23 (7H).

### Example 9

### <Synthesis of ([1,1':4',1"]terphenyl-4-yl)-(4-naphthalen-1-yl-phenyl)-[1,1':2',1":4",1‴]quaterphenyl-4'-yl-amine (1-27)>

To a reaction vessel purged with nitrogen, 9.2 g of (4-naphthalen-1-yl-phenyl)-[1,1':4',1"]terphenyl-3-yl-amine, 5.8 g of 4-bromo-[1,1':4',1"]terphenyl, 2.2 g of sodium tert-butoxide, and 90 mL of toluene were added. To the vessel, 0.1 g of tris(dibenzylideneacetone)dipalladium(0) and 0.2 g of a 50% toluene solution of tri(tert-butylphosphine) were added, and the mixture was heated and stirred under reflux for 4 hours. After the mixture was cooled to 80°C, the inorganic substances were removed by hot filtration, and then, the filtrate was concentrated. To the residue, 130 mL of toluene was added, and the mixture was stirred while heating. Then, 6 g of silica gel and 6 g of activated clay were added thereto at 80°C, and the mixture was stirred for 1 hour. The solid was removed by hot filtration, and the filtrate was concentrated. The residue was recrystallized using dichloromethane and acetone, whereby 11.4 g of a white solid of ([1,1':4',1"]terphenyl-4-yl)-(4-naphthalen-1-yl-phenyl)-[1,1':4',1"]terphenyl-3-yl-amine (yield: 89.8%) was obtained.

To a reaction vessel purged with nitrogen, 11.4 g of the obtained ([1,1':4',1"]terphenyl-4-yl)-(4-naphthalen-1-yl-phenyl)-[1,1':4',1"]terphenyl-3-yl-amine was added, and subsequently, 114 mL of dimethylformamide was added thereto, and the mixture was cooled to 0°C. After 3.0 g of N-bromosuccinimide was slowly added thereto and the mixture was stirred at 0°C for 1 hour, the temperature was slowly raised to room temperature, and the mixture was stirred for 3 hours. The reaction solution was added to 360 mL of water, and the precipitated solid was collected by filtration. To the solid, 130 mL of toluene was added, and the mixture was stirred while heating. Then, 6 g of silica gel was added thereto at 80°C, and the mixture was stirred for 1 hour. The solid was removed by hot filtration, and the filtrate was concentrated. The residue was recrystallized using dichloromethane and acetone, whereby 8.7 g of a white solid of ([1,1':4',1"]terphenyl-4-yl)-(4-naphthalen-1-yl-phenyl)-(6-bromo-[1,1':4',1"]terphenyl-3-yl)amine (yield: 69%) was obtained.

To a reaction vessel purged with nitrogen, 8.7 g of the obtained ([1,1:4',1"]terphenyl-4-yl)-(4-naphthalen-1-yl-phenyl)-(6-bromo-[1,1':4',1"]terphenyl-3-yl)amine was added, and subsequently, 1.6 g of phenylboronic acid, 1.5 g of sodium bicarbonate, 100 mL of tetrahydrofuran, and 35 mL of water were added. To the vessel, 0.2 g of diphenylphosphino ferrocene palladium dichloride was added, and the mixture was stirred under reflux for 12 hours. After the mixture was cooled, extraction was performed using ethyl acetate, and the organic layer was concentrated. To the residue, 100 mL of toluene was added, and the mixture was stirred while heating. Thereafter, 5 g of silica gel was added thereto at 80°C, and the mixture was stirred for 1 hour, and then, the solid was removed by hot filtration. The filtrate was concentrated, and the residue was repeatedly recrystallized twice using dichloromethane and acetone, whereby 6.8 g of a white solid of ([1,1':4',1"]terphenyl-4-yl)-(4-naphthalen-1-yl-phenyl)-[1,1':2',1":4",1‴]quaterphenyl-4'-yl-amine (1-27) (yield: 78%) was obtained.

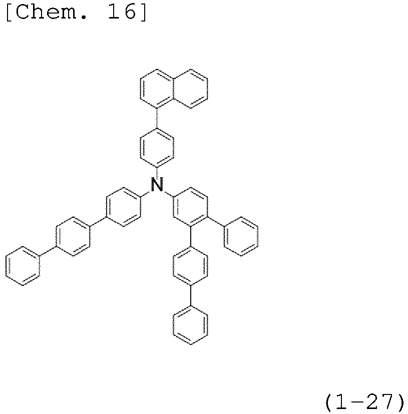

The structure of the obtained white solid was identified using NMR.

The following 41 hydrogen signals were detected by ¹H-NMR (CDCl₃).
δ (ppm) = 8.04-8.06 (1H), 7.89-7.91 (1H), 7.83-7.85 (1H), 7.60-7.70 (8H), 7.27-7.55 (23H), 7.18-7.23 (7H).

### Example 10

### <Synthesis of (biphenyl-4-yl)-(4'-triphenylsilyl-biphenyl-4-yl)-[1,1':2',1"]terphenyl-4'-yl-amine (1-62)>

To a reaction vessel, 20.0 g of 4-bromotriphenylsilane, 9.0 g of 4-chlorophenylboronic acid, 10.0 g of potassium carbonate, 160 mL of toluene, 80 mL of ethanol, and 60 mL of water were added. To the vessel, 1.1 g of tetrakistriphenylphosphine palladium was added, and the mixture was stirred under reflux for 12 hours. After cooling, the mixture was separated, and the organic layer was washed with water and then with saturated saline, and dried over anhydrous magnesium sulfate. After the desiccant was removed by filtration and the filtrate was stirred while heating, 10 g of silica gel was added thereto at 80°C. The mixture was stirred for 1 hour, and the solid was removed by hot filtration, and then, the filtrate was concentrated. The residue was recrystallized using toluene and heptane, whereby 12.7 g of a yellowish white solid of 4-chloro-4'-triphenylsilyl-biphenyl (yield: 59.0%) was obtained.

To a reaction vessel purged with nitrogen, 6.0 g of the obtained 4-chloro-4'-triphenylsilyl-biphenyl was added together with 6.6 g of (biphenyl-4-yl)-[1,1':2',1"]terphenyl-4-yl-amine, and subsequently, 2.6 g of sodium tert-butoxide and 60 mL of toluene were added. To the vessel, 0.14 g of bis[tri(tert-butylphosphine)]palladium was added, and the mixture was heated and stirred under reflux for 3 hours. After the mixture was cooled to 80°C, 6 g of silica gel was added to the reaction solution, and the mixture was stirred for 30 minutes, and then, the solid was removed by hot filtration. The filtrate was concentrated, and the residue was purified by column chromatography (silica gel: 200 g; heptane : dichloromethane = 3:1), whereby 3.5 g of a white solid of (biphenyl-4-yl)-(4'-triphenylsilyl-biphenyl-4-yl)-[1,1':2',1"]terphenyl-4'-yl-amine (1-62) (yield: 30%) was obtained.

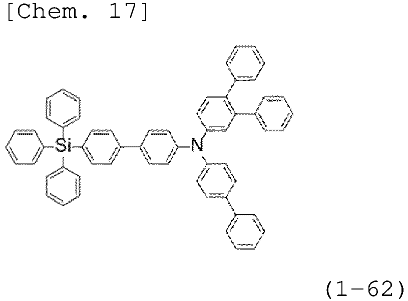

The structure of the obtained white solid was identified using NMR.

The following 47 hydrogen signals were detected by ¹H-NMR (CDCl₃).
δ (ppm) = 8.03 (1H), 7.83-7.90 (3H), 7.72-7.75 (1H), 7.55-7.67 (14H), 7.26-7.47 (17H), 7.07-7.23 (11H).

### Example 11

### <Synthesis of ([1,1':2',1"]terphenyl-4'-yl)-(4-naphthalen-1-yl-phenyl)-[1,1':4',1":4",1‴]quaterphenyl-4-yl-amine (1-68) >

To a reaction vessel purged with nitrogen, 25.0 g of (4-naphthalen-1-yl-phenyl)-[1,1':2',1"]terphenyl-4'-yl-amine, 17.4 g of 1-bromo-4-iodobenzene, 7.7 g of sodium tert-butoxide, and 375 mL of toluene were added. To the vessel, 0.13 g of palladium acetate and 0.32 g of 4,5'-bis(diphenylphosphino)-9,9-dimethylxanthene were added, and the mixture was heated and stirred under reflux for 14 hours. The mixture was cooled to 80°C, and the inorganic substances were removed by hot filtration, and then, the filtrate was concentrated. The residue was purified by column chromatography (silica gel: 200 g; heptane : dichloromethane = 3:1), whereby 28.0 g of a yellowish white solid of (4-bromophenyl)-(4-naphthalen-1-yl-phenyl)-[1,1':2',1"]terphenyl-4'-yl-amine (yield: 83.1%) was obtained.

To a reaction vessel, 8.0 g of the obtained (4-bromophenyl)-(4-naphthalen-1-yl-phenyl)-[1,1':2',1"]terphenyl-4'-yl-amine was added, and subsequently, 5.2 g of 2-([1,1':4',1"]terphenyl-4-yl)-4,4,5,5-tetramethyl-1,3,2-dioxaborolane, 1.7 g of sodium bicarbonate, 96 mL of tetrahydrofuran, and 32 mL of water were added. To the vessel, 0.22 g of diphenylphosphino ferrocene palladium dichloride was added, and the mixture was stirred under reflux for 4 hours. After the mixture was cooled, extraction was performed using ethyl acetate, and the organic layer was washed with water and then with saturated saline, and dried over anhydrous magnesium sulfate. The desiccant was removed by filtration, and the filtrate was concentrated. To the residue, 100 mL of toluene was added, and then, 5 g of silica gel was added thereto at 80°C. After the mixture was stirred for 1 hour, the solid was removed by hot filtration and then, the filtrate was concentrated. The residue was recrystallized using tetrahydrofuran and acetone, whereby 4.6 g of a yellowish white solid of ([1,1:2',1"]terphenyl-4'-yl)-(4-naphthalen-1-yl-phenyl)-[1,1':4',1":4",1‴]quaterphenyl-4-yl-amine (1-67) (yield: 46%) was obtained.

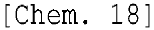

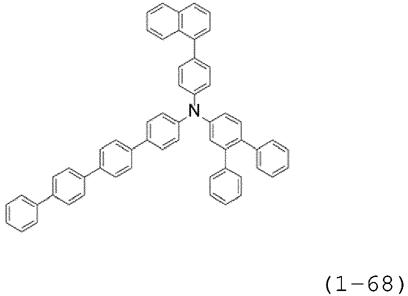

The structure of the obtained yellowish white solid was identified using NMR.

The following 41 hydrogen signals were detected by ¹H-NMR (CDCl₃).
δ (ppm) = 8.04-8.06 (1H), 7.83-7.91 (2H), 7.60-7.73 (12H), 7.43-7.52 (8H), 7.13-7.38 (18H).

### Example 12

### <Synthesis of compound (2-11)>

To a reaction vessel, 45.0 g of 1-bromobenzene (D-substituted), 58.0 g of 4-tert-butylaniline, 1.0 g of palladium(II) acetate, 30.0 g of sodium tert-butoxide, 2.0 g of bis(diphenylphosphino)-1,1'-binaphthyl, and 450 mL of toluene were added, and the mixture was stirred under reflux for 24 hours. After being allowed to cool, the mixture was concentrated and purified by column chromatography, whereby 49.9 g of a powder of the following compound (2-11a) (yield: 78%) was obtained.

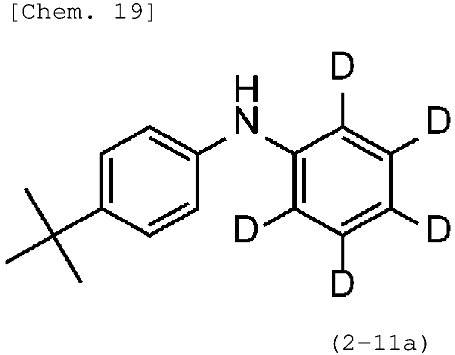

To a reaction vessel, 20.0 g of the above compound (2-11a), 18.4 g of the following compound (2-11b), 0.5 g of palladium(II) acetate, 18.9 g of sodium tert-butoxide, 0.8 g of tri(tert-butyl)phosphine, and 200 mL of toluene were added, and the mixture was stirred under reflux for 24 hours. After being allowed to cool, the mixture was concentrated and purified by column chromatography, whereby 21.5 g of a powder of the following compound (2-11c) (yield: 84%) was obtained.

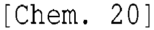

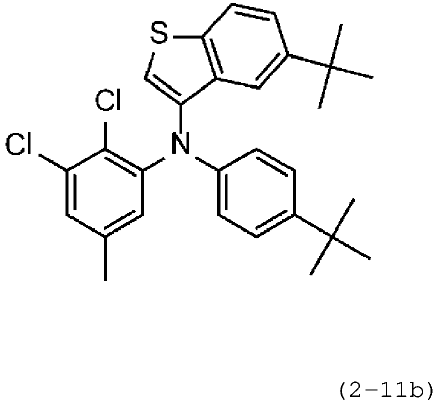

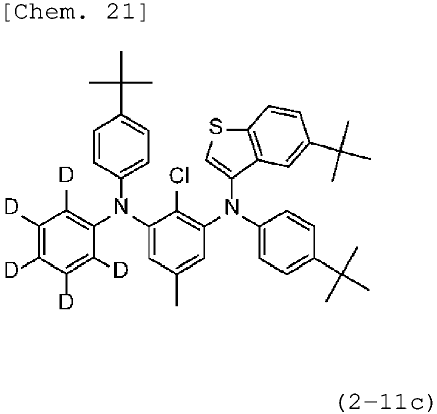

To a reaction vessel, 12.0 g of the above compound (2-11c) and 120 mL of tert-butylbenzene were added, and then, 42.5 mL of n-butyllithium was added thereto dropwise at - 78°C, and the mixture was aerated with nitrogen gas while stirring at 60°C for 3 hours. Subsequently, 11.3 g of boron tribromide was added thereto dropwise at -78°C, and thereafter, the mixture was stirred at normal temperature for 1 hour. Then, 5.9 g of N,N-diisopropylethylamine was further added thereto dropwise at 0°C, and the mixture was stirred at 120°C for 2 hours. After the mixture was allowed to cool, an aqueous sodium acetate solution was added thereto, and the mixture was stirred. Extraction was performed with ethyl acetate, and the organic layer was concentrated, and then, the concentrate was purified by column chromatography, whereby 1.7 g of a powder of the following compound (2-11) (yield: 11%) was obtained.

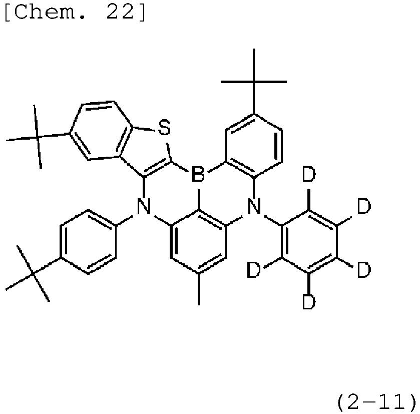

### Example 13

The glass transition points (Tg) of the arylamine compounds represented by the general formula (1) were measured using a high-sensitive differential scanning calorimeter (DSC3100SA manufactured by Bruker AXS). The measurement results are shown below.

| | Glass transition point (Tg) |
|---|---|
| Compound (1-13) of Example 1 | 111.1°C |
| Compound (1-17) of Example 2 | 109.7°C |
| Compound (1-30) of Example 3 | 123.7°C |
| Compound (1-32) of Example 4 | 121.0°C |
| Compound (1-41) of Example 5 | 125.2°C |
| Compound (1-44) of Example 6 | 118.7°C |
| Compound (1-58) of Example 7 | 120.7°C |
| Compound (1-14) of Example 8 | 120.4°C |
| Compound (1-27) of Example 9 | 123.3°C |
| Compound (1-62) of Example 10 | 124.0°C |
| Compound (1-68) of Example 11 | 120.6°C |

The arylamine compounds represented by the general formula (1) have a glass transition point (Tg) of 100°C or higher, which shows that the compounds are stable in a thin film state.

### Example 14

Vapor-deposited films with a film thickness of 100 nm were fabricated on ITO substrates using the arylamine compounds represented by the general formula (1), and work functions were measured using an ionization potential measuring device (PYS-202 manufactured by Sumitomo Heavy Industries, Ltd.). The measurement results are shown below.

| | Work function |
|---|---|
| Compound (1-13) of Example 1 | 5.69 eV |
| Compound (1-17) of Example 2 | 5.65 eV |
| Compound (1-30) of Example 3 | 5.69 eV |
| Compound (1-32) of Example 4 | 5.75 eV |
| Compound (1-41) of Example 5 | 5.69 eV |
| Compound (1-44) of Example 6 | 5.71 eV |
| Compound (1-58) of Example 7 | 5.75 eV |
| Compound (1-14) of Example 8 | 5.71 eV |
| Compound (1-27) of Example 9 | 5.72 eV |
| Compound (1-62) of Example 10 | 5.71 eV |
| Compound (1-68) of Example 11 | 5.69 eV |

The arylamine compounds represented by the general formula (1) have a favorable energy level compared to the work function 5.4 eV of common hole transport materials such as NPD and TPD, and therefore are found to have a good hole transport ability and an excellent electron blocking ability.

### Example 15

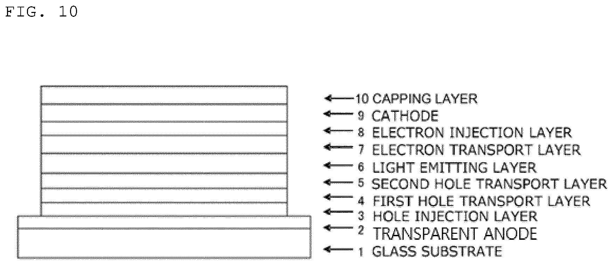

The organic EL device was fabricated by vapor-depositing a hole injection layer 3, a first hole transport layer 4, a second hole transport layer 5, a light emitting layer 6, an electron transport layer 7, an electron injection layer 8, a cathode 9, and a capping layer 10 in this order on a reflective ITO electrode formed beforehand as a transparent anode 2 on a glass substrate 1 as shown in FIG. 10.

Specifically, as the transparent anode 2, ITO with a film thickness of 50 nm, a silver alloy reflective film with a film thickness of 100 nm, and ITO with a film thickness of 5 nm were sequentially deposited on the glass substrate 1, and after ultrasonic washing was performed in isopropyl alcohol for 20 minutes, the film was dried for 10 minutes on a hot plate heated to 250°C. Thereafter, a UV ozone treatment was performed for 15 minutes, and then, the glass substrate with ITO was fixed in a vacuum vapor deposition apparatus, and the pressure was reduced to 0.001 Pa or less. Subsequently, as the hole injection layer 3, an electron acceptor (Acceptor-1) of the following structural formula and a compound (HTM-1) of the following structural formula were formed to a film thickness of 10 nm by binary vapor deposition at such a vapor deposition rate that the vapor deposition rate ratio is as follows: Acceptor-1 : the compound (HTM-1) = 3:97 so as to cover the transparent anode 2. On the hole injection layer 3, as the first hole transport layer 4, the compound (HTM-1) of the following structural formula was formed to a film thickness of 140 nm. On the first hole transport layer 4, as the second hole transport layer 5, the compound (1-13) of Example 1 was formed to a film thickness of 5 nm. On the second hole transport layer 5, as the light emitting layer 6, the compound (2-11) of Example 12 and a compound (EMH-1) of the following structural formula were formed to a film thickness of 20 nm by binary vapor deposition at such a vapor deposition rate that the vapor deposition rate ratio is as follows: the compound (2-11) : the compound (EMH-1) = 5:95. On the light emitting layer 6, as the electron transport layer 7, a compound (ETM-1) of the following structural formula and a compound (ETM-2) of the following structural formula were formed to a film thickness of 30 nm by binary vapor deposition at such a vapor deposition rate that the vapor deposition rate ratio was as follows: the compound (ETM-1) : the compound (ETM-2) = 50:50. On the electron transport layer 7, as the electron injection layer 8, lithium fluoride was formed to a film thickness of 1 nm. On the electron injection layer 8, as the cathode 9, a magnesium-silver alloy was formed to a film thickness of 12 nm. Finally, as the capping layer 10, a compound (CPL-1) of the following structural formula was formed to a film thickness of 60 nm. The emission characteristics of the fabricated organic EL device when applying a DC voltage in the air at normal temperature were measured. The results are summarized in Table 1.

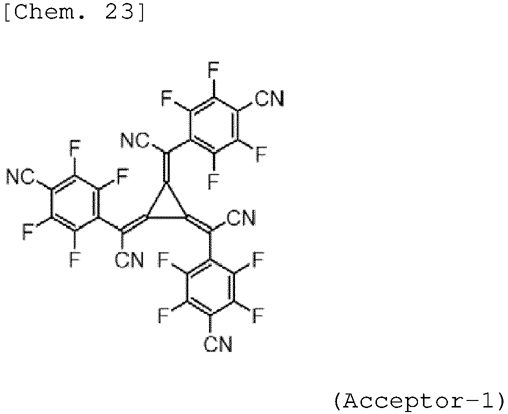

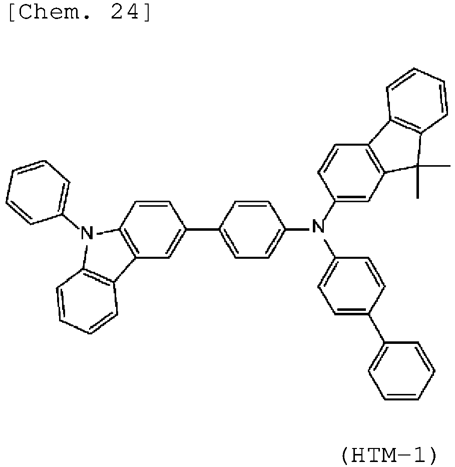

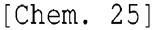

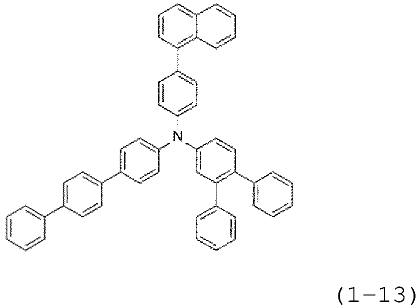

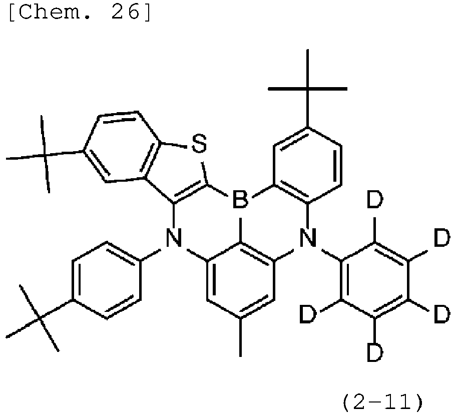

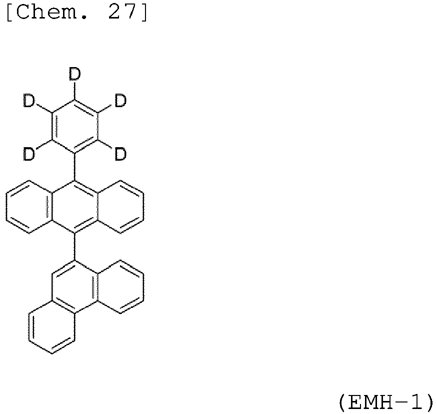

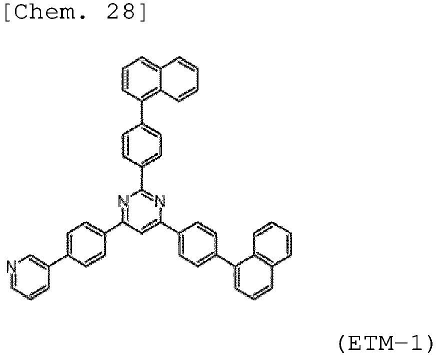

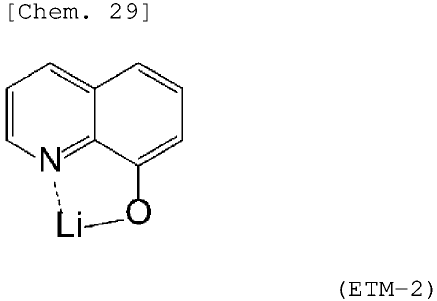

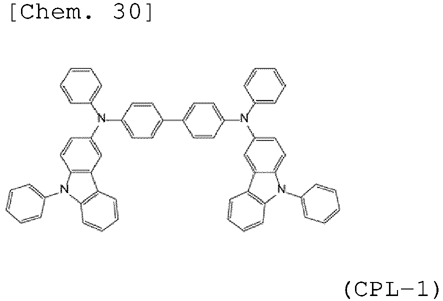

### Example 16

An organic EL device was fabricated under the same conditions as in Example 15 except that as the material of the second hole transport layer 5, the compound (1-17) of Example 2 was used in place of the compound (1-13) of Example 1. The characteristics of the fabricated organic EL device were measured in the air at normal temperature. The measurement results of the emission characteristics when applying a DC voltage to the fabricated organic EL device are summarized in Table 1.

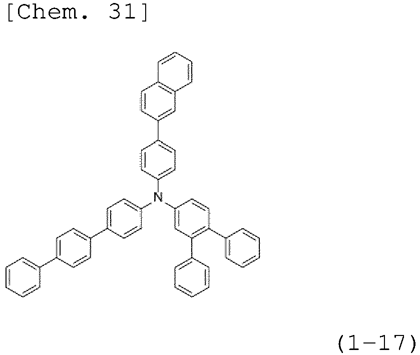

### Example 17

An organic EL device was fabricated under the same conditions as in Example 15 except that as the material of the second hole transport layer 5, the compound (1-30) of Example 3 was used in place of the compound (1-13) of Example 1. The characteristics of the fabricated organic EL device were measured in the air at normal temperature. The measurement results of the emission characteristics when applying a DC voltage to the fabricated organic EL device are summarized in Table 1.

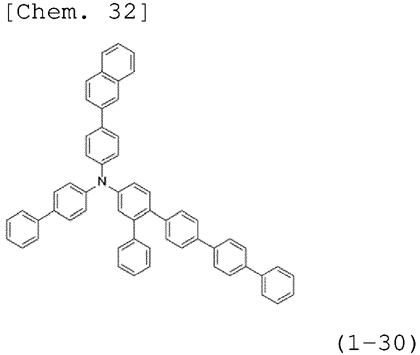

### Example 18

An organic EL device was fabricated under the same conditions as in Example 15 except that as the material of the second hole transport layer 5, the compound (1-32) of Example 4 was used in place of the compound (1-13) of Example 1. The characteristics of the fabricated organic EL device were measured in the air at normal temperature. The measurement results of the emission characteristics when applying a DC voltage to the fabricated organic EL device are summarized in Table 1.

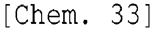

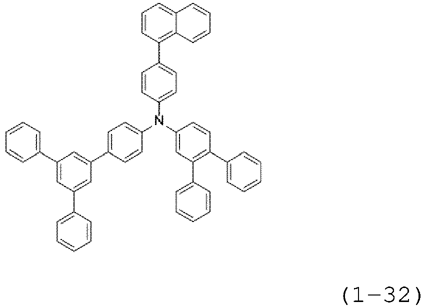

### Example 19

An organic EL device was fabricated under the same conditions as in Example 15 except that as the material of the second hole transport layer 5, the compound (1-41) of Example 5 was used in place of the compound (1-13) of Example 1. The characteristics of the fabricated organic EL device were measured in the air at normal temperature. The measurement results of the emission characteristics when applying a DC voltage to the fabricated organic EL device are summarized in Table 1.

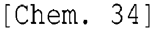

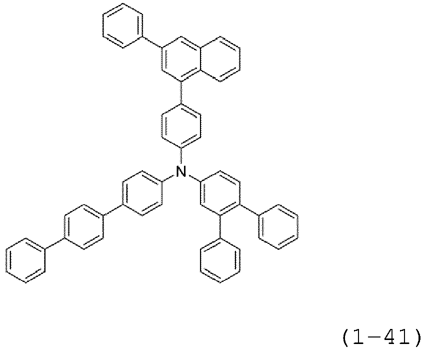

### Example 20

An organic EL device was fabricated under the same conditions as in Example 15 except that as the material of the second hole transport layer 5, the compound (1-44) of Example 6 was used in place of the compound (1-13) of Example 1. The characteristics of the fabricated organic EL device were measured in the air at normal temperature. The measurement results of the emission characteristics when applying a DC voltage to the fabricated organic EL device are summarized in Table 1.

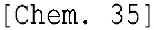

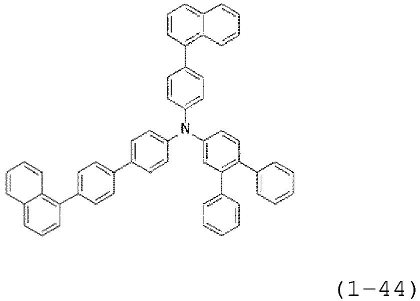

### Example 21

An organic EL device was fabricated under the same conditions as in Example 15 except that as the material of the second hole transport layer 5, the compound (1-58) of Example 7 was used in place of the compound (1-13) of Example 1. The characteristics of the fabricated organic EL device were measured in the air at normal temperature. The measurement results of the emission characteristics when applying a DC voltage to the fabricated organic EL device are summarized in Table 1.

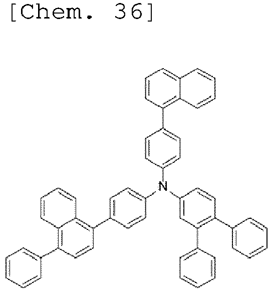

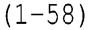

### Example 22

An organic EL device was fabricated under the same conditions as in Example 15 except that as the material of the second hole transport layer 5, the compound (1-14) of Example 7 was used in place of the compound (1-13) of Example 1. The characteristics of the fabricated organic EL device were measured in the air at normal temperature. The measurement results of the emission characteristics when applying a DC voltage to the fabricated organic EL device are summarized in Table 1.

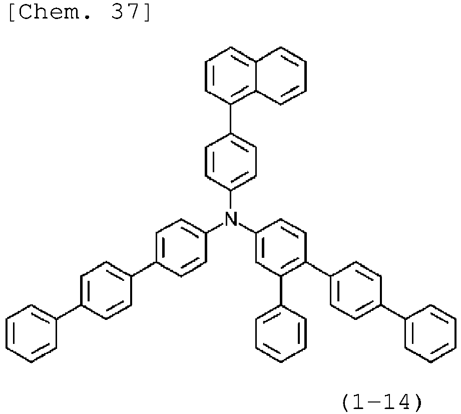

### Example 23

An organic EL device was fabricated under the same conditions as in Example 15 except that as the material of the second hole transport layer 5, the compound (1-27) of Example 7 was used in place of the compound (1-13) of Example 1. The characteristics of the fabricated organic EL device were measured in the air at normal temperature. The measurement results of the emission characteristics when applying a DC voltage to the fabricated organic EL device are summarized in Table 1.

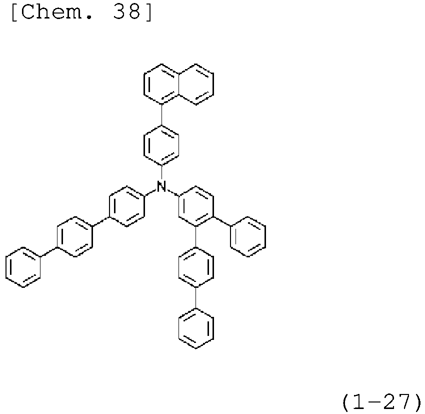

### Example 24

An organic EL device was fabricated under the same conditions as in Example 15 except that as the material of the second hole transport layer 5, the compound (1-62) of Example 7 was used in place of the compound (1-13) of Example 1. The characteristics of the fabricated organic EL device were measured in the air at normal temperature. The measurement results of the emission characteristics when applying a DC voltage to the fabricated organic EL device are summarized in Table 1.

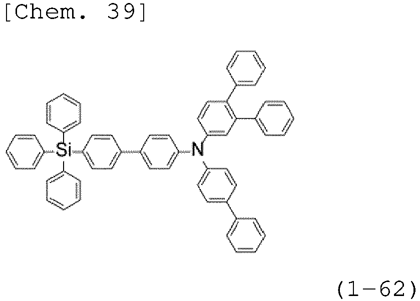

### Example 25

An organic EL device was fabricated under the same conditions as in Example 15 except that as the material of the second hole transport layer 5, the compound (1-68) of Example 7 was used in place of the compound (1-13) of Example 1. The characteristics of the fabricated organic EL device were measured in the air at normal temperature. The measurement results of the emission characteristics when applying a DC voltage to the fabricated organic EL device are summarized in Table 1.

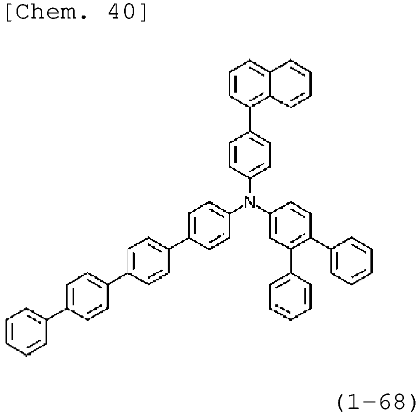

### [Comparative Example 1]

For comparison, an organic EL device was fabricated under the same conditions as in Example 15 except that as the material of the second hole transport layer 5, a compound (HTM-2) of the following structural formula was used in place of the compound (1-13) of Example 1. The characteristics of the fabricated organic EL device were measured in the air at normal temperature. The measurement results of the emission characteristics when applying a DC voltage to the fabricated organic EL device are summarized in Table 1.

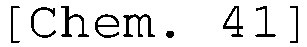

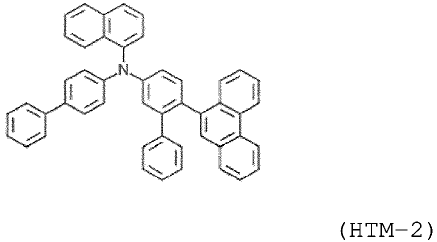

### [Comparative Example 2]

For comparison, an organic EL device was fabricated under the same conditions as in Example 15 except that as the material of the second hole transport layer 5, a compound (HTM-3) of the following structural formula was used in place of the compound (1-13) of Example 1. The characteristics of the fabricated organic EL device were measured in the air at normal temperature. The measurement results of the emission characteristics when applying a DC voltage to the fabricated organic EL device are summarized in Table 1.

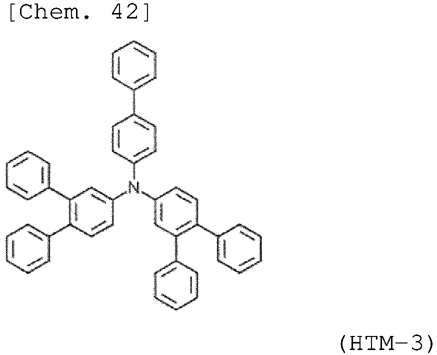

### [Comparative Example 3]

For comparison, an organic EL device was fabricated under the same conditions as in Example 15 except that as the material of the second hole transport layer 5, a compound (HTM-4) of the following structural formula was used in place of the compound (1-13) of Example 1. The characteristics of the fabricated organic EL device were measured in the air at normal temperature. The measurement results of the emission characteristics when applying a DC voltage to the fabricated organic EL device are summarized in Table 1.

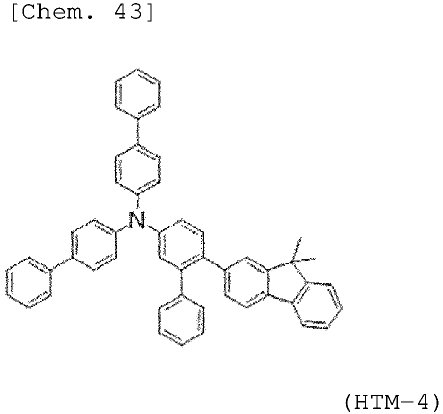

The device lifetime was measured using the organic EL devices fabricated in Examples 15 to 25 and Comparative Examples 1 to 3. The results are summarized in Table 1. The device lifetime was measured as a time elapsed until the emission luminance was attenuated to 1,900 cd/m² (corresponding to 95% when the initial luminance was taken as 100%: attenuation to 95%) when constant current driving was performed by setting the emission luminance at the start of emission (initial luminance) to 2,000 cd/m².

**[Table 1]**

| | Second hole transport layer | (@10mA/cm²) | | | | Device lifetime |
|---|---|---|---|---|---|---|
| | | Voltage[V] | Luminance [cd/m²] | Luminous efficiency [cd/A] | Power efficiency [lm/W] | Attenuation to 95% |
| Example 15 | Compound (1-13) | 3. 43 | 1055 | 10. 56 | 9. 67 | 535h |
| Example 16 | Compound (1-17) | 3. 40 | 968 | 9. 68 | 8.94 | 51 5h |
| Example 17 | Compound (1-30) | 3. 39 | 996 | 9. 96 | 9. 23 | 505h |
| Example 18 | Compound (1-32) | 3. 44 | 1006 | 10. 09 | 9. 20 | 345h |
| Example 19 | Compound (1-41) | 3. 44 | 1066 | 10. 68 | 9.75 | 457h |
| Example 20 | Compound (1-44) | 3. 44 | 1049 | 10.51 | 9. 61 | 457h |
| Example 21 | Compound (1-58) | 3. 48 | 1067 | 10. 68 | 9. 45 | 406h |
| Example 22 | Compound (1-14) | 3. 43 | 1076 | 10. 75 | 9. 78 | 462h |
| Example 23 | Compound (1-27) | 3. 44 | 1011 | 10.11 | 9. 20 | 627h |
| Example 24 | Compound (1-62) | 3. 42 | 988 | 9. 89 | 9. 00 | 477h |
| Example 25 | Compound (1-68) | 3. 41 | 1038 | 10. 32 | 9. 58 | 421h |
| Comparative Example1 | HTM-2 | 3. 52 | 934 | 9. 34 | 8. 61 | 335h |
| Comparative Example2 | HTM-3 | 3. 55 | 794 | 7. 94 | 7. 30 | 323h |
| Comparative Example3 | HTM-4 | 3.71 | 882 | 8.83 | 8.03 | 306h |

As shown in Table 1, the luminous efficiency when passing a current with a current density of 10 mA/cm² was 9.68 to 10.75 cd/A in the organic EL devices of Examples 15 to 25, which was higher than 7.94 to 9.34 cd/A in the organic EL devices of Comparative Examples 1 to 3. Further, the power efficiency was 8.94 to 9.78 lm/W in the organic EL devices of Examples 15 to 25, which was also higher than 7.30 to 8.61 lm/W in the organic EL devices of Comparative Examples 1 to 3. Further, the device lifetime (attenuation to 95%) was 345 to 627 hours in the organic EL devices of Examples 15 to 25, which is found to be significantly increased compared to 306 to 335 hours in the organic EL devices of Comparative Examples 1 to 3.

As is clear from the above results, the arylamine compound having a specific structure represented by the general formula (1) according to the invention has larger hole mobility and a superior electron blocking ability compared to a conventional arylamine compound used as a hole transport material, and therefore, it was found that the organic EL device used together with the blue light emitting layer of the invention can achieve an organic EL device having a high luminous efficiency and a long lifetime compared to a conventional organic EL device.

### Industrial Applicability

In the organic EL device, in which an arylamine compound having a specific structure is used, of the invention, the durability of the organic EL device can be improved as well as improving the luminous efficiency, and for example, it has become possible to develop applications to home electric appliances and illuminations.

### Reference Signs List

- 1: Glass substrate
- 2: Transparent anode
- 3: Hole injection layer
- 4: First hole transport layer
- 5: Second hole transport layer
- 6: Light emitting layer
- 7: Electron transport layer
- 8: Electron injection layer
- 9: Cathode
- 10: Capping layer

## Claims

1. An organic electroluminescent device comprising at least an anode, a first hole transport layer, a second hole transport layer, a blue light emitting layer, an electron transport layer, and a cathode in this order, **characterized in that** the second hole transport layer contains an arylamine compound represented by the following general formula (1): 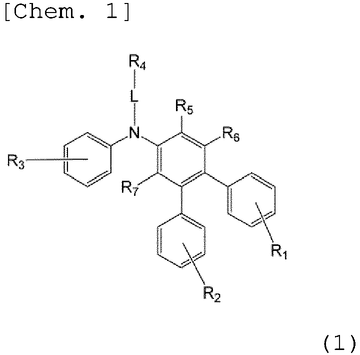 wherein R₁ to R₄ may be the same or different, and represent a hydrogen atom, a deuterium atom, a carbonyl group, a cyano group, a silyl group which may have a substituent, a phosphino group which may have a substituent, a phosphine oxide group which may have a substituent, a linear or branched alkyl group having 1 to 6 carbon atoms which may have a substituent, or a substituted or unsubstituted aromatic hydrocarbon group having 6 to 25 ring-forming carbon atoms; L represents a divalent group of a substituted or unsubstituted aromatic hydrocarbon having 6 to 20 ring-forming carbon atoms or a single bond; and R₅ to R₇ represent a hydrogen atom, a deuterium atom, a linear or branched alkyl group having 1 to 6 carbon atoms which may have a substituent, a halogen atom, or a cyano group.

2. The organic electroluminescent device according to claim 1, **characterized in that** in the general formula (1), R₁ and R₂ may be the same or different, a hydrogen atom, a silyl group which may have a substituent, or a substituted or unsubstituted aromatic hydrocarbon group having 6 to 20 ring-forming carbon atoms; R₃ may be a silyl group which may have a substituent or a substituted or unsubstituted aromatic hydrocarbon group having 6 to 25 ring-forming carbon atoms; and R₄ may be a hydrogen atom, a silyl group which may have a substituent, or a substituted or unsubstituted aromatic hydrocarbon group having 6 to 25 ring-forming carbon atoms.

3. The organic electroluminescent device according to claim 1 or 2, **characterized in that** in the general formula (1), R₁ and R₂ may be the same or different, any of a hydrogen atom, an unsubstituted phenyl group, an unsubstituted naphthyl group, an unsubstituted biphenylyl group, a phenyl group substituted with a naphthyl group, and an unsubstituted terphenylyl group; R₃ may be any of an unsubstituted phenyl group, an unsubstituted biphenylyl group, a phenyl group substituted with a naphthyl group, a phenyl group substituted with two phenyl groups, and a naphthyl group substituted with a phenyl group; and R₄ may be any of an unsubstituted phenyl group, an unsubstituted naphthyl group, and a naphthyl group substituted with a phenyl group.

4. The organic electroluminescent device according to any one of claims 1 to 3, **characterized in that** in the general formula (1), R₁ may be any of an unsubstituted phenyl group, an unsubstituted biphenylyl group, a phenyl group substituted with a naphthyl group, and an unsubstituted terphenylyl group; R₂ may be any of a hydrogen atom, an unsubstituted phenyl group, an unsubstituted naphthyl group, an unsubstituted biphenylyl group, a phenyl group substituted with a naphthyl group, and an unsubstituted terphenylyl group; R₃ is an unsubstituted phenyl group; and R₄ may be any of an unsubstituted phenyl group, an unsubstituted naphthyl group, and a naphthyl group substituted with a phenyl group.

5. The organic electroluminescent device according to claim 1 or 2, **characterized in that** in the general formula (1), at least one of R₁ to R₄ is either a triphenylsilyl group or a phenyl group substituted with a triphenylsilyl group.

6. The organic electroluminescent device according to any one of claims 1 to 5, **characterized in that** in the general formula (1), all of R₅ to R₇ are a hydrogen atom.

7. The organic electroluminescent device according to any one of claims 1 to 6, **characterized in that** in the general formula (1), L is a 1,4-phenylene group.

8. The organic electroluminescent device according to any one of claims 1 to 7, **characterized in that** the blue light emitting layer contains a pyrene derivative having a pyrene skeleton in the molecule as a blue light emitting dopant.

9. The organic electroluminescent device according to any one of claims 1 to 7, **characterized in that** the blue light emitting layer contains a compound represented by the following general formula (2) or general formula (3) as a blue light emitting dopant: 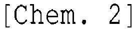 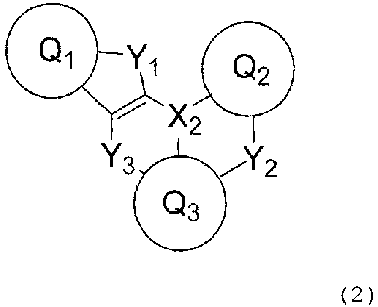 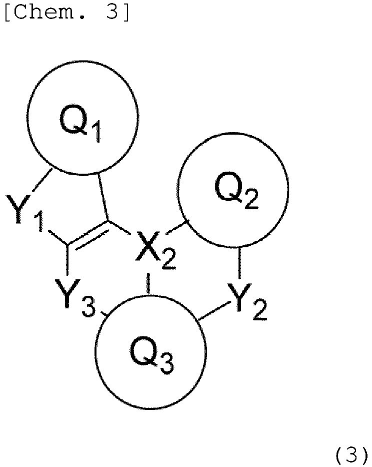 Wherein the general formula (2) and general formula (3), Q₁ to Q₃ may be the same or different, and represent a substituted or unsubstituted aromatic hydrocarbon or a substituted or unsubstituted aromatic heterocyclic ring; X₂ represents B, P, P=O, or P=S; Y₁ to Y₃ may be the same or different, and represent any one selected from N-R₈, CR₉R₁₀, O, S, Se, and SiR₁₁R₁₂, R₈ to R₁₂ may be the same or different, and represent a hydrogen atom, a deuterium atom, a fluorine atom, a chlorine atom, a cyano group, a nitro group, a linear or branched alkyl group having 1 to 6 carbon atoms which may have a substituent, a cycloalkyl group having 5 to 10 carbon atoms which may have a substituent, a linear or branched alkenyl group having 2 to 6 carbon atoms which may have a substituent, a linear or branched alkyloxy group having 1 to 6 carbon atoms which may have a substituent, a cycloalkyloxy group having 5 to 10 carbon atoms which may have a substituent, a substituted or unsubstituted aromatic hydrocarbon group, a substituted or unsubstituted aromatic heterocyclic group, or a substituted or unsubstituted aryloxy group, R₉ and R₁₀, and R₁₁ and R₁₂ may bind to each other to form a ring via a single bond, a substituted or unsubstituted methylene group, an oxygen atom, a sulfur atom, or a monosubstituted amino group, provided that when Y₁ to Y₃ are N-R₈, CR₉R₁₀, or SiR₁₁R₁₂, R₈ to R₁₂ may each bind to the adjacent Q₁ to Q₃ to form a ring via a single bond or a linking group such as a substituted or unsubstituted methylene group, an oxygen atom, a sulfur atom, or a monosubstituted amino group.

10. The organic electroluminescent device according to any one of claims 1 to 9, **characterized in that** the blue light emitting layer contains an anthracene derivative having an anthracene skeleton in the molecule.
